# EUROPEAN PATENT APPLICATION

(11) **EP 1 538 165 A1**
(43) Date of publication of application: **08.06.2005**
(21) Application number: 03027772.7
(22) Date of filing: 03.12.2003
(51) Int. Cl.: C07K 16/28, A61K 39/395, A61P 9/10, C12N 5/20, C12N 15/13

(54) **Inhibitors of glycoprotein VI based on monoclonal antibody hgp 5c4**

(71) Applicant: Procorde GmbH, 82152 Martinsried (DE); GSF-Forschungszentrum für Umwelt und Gesundheit GmbH, 85764 Oberschleissheim (DE)
(72) Inventor: Gawaz, Meinrad, 80805 München (DE); Kremmer, Elisabeth, 95354 Freising (DE); Münch, Götz, 80799 München (DE)
(74) Representative: Hartz, Nikolai F., Dr.

(57) **Abstract**

The present invention relates to a specific inhibitor of human platelet glycoprotein VI. Moreover, the present invention relates to pharmaceutical compositions containing the specific inhibitor of the invention. The present invention also relates to hybridomas expressing the antibody of the present invention. Furthermore, the present invention relates to the use of the specific inhibitor of glycoprotein VI for the preparation of a medicament for the prevention of acute and chronic vascular diseases associated with arterial and/or intravenous thrombosis, such as acute coronary syndrome, acute carotid artery syndrome, acute myocardial infarction, acute cerebral stroke and chronic coronary or peripheral vessel disease. Finally, the present invention relates to a process for preparing the antibody of the invention and to a process for preparing a specific fragment of the antibody of the present invention.

SEQUENCE LISTING

<110>  ProCorde GmbH

<120>  Inhibitors of Glycoprotein VI based on Monoclonal Antibody hGP 5C4

<130>  EAD-12518

<140>  EP 03027772.7
<141>  2003-12-03

<160>  7

<170>  PatentIn version 3.1

<210>  1
<211>  504
<212>  PRT
<213>  Artificial Sequence

<220>
<223>  fusion protein

<400>  1

<210> 2
<211> 37
<212> DNA
<213> Artificial Sequence

<220>
<223> PCR primer

<400> 2
cgcggggcgg ccgcgagtcc aaatcttgtg acaaaac 37

<210> 3
<211> 33
<212> DNA
<213> Artificial Sequence

<220>
<223> PCR primer

<400> 3
gcgggaagct ttcatttacc cggagacagg gag 33

<210> 4
<211> 36
<212> DNA
<213> Artificial Sequence

<220>
<223> PCR primer'

<400> 4
gcggggagat ctaccaccat gtctccatcc ccgacc 36

<210> 5
<211> 34
<212> DNA

<213>  Artificial Sequence

<220>
<223>  PCR primer

<400>  5
cgcggggcgg ccgccgttgc ccttggtgta gtac                                    34


<210>  6
<211>  35
<212>  DNA
<213>  Artificial Sequence

<220>
<223>  PCR primer

<400>  6
gcgggggcta gcaccaccat gtctccatcc ccgac                                   35


<210>  7
<211>  34
<212>  DNA
<213>  Artificial Sequence

<220>
<223>  PCR primer

<400>  7
cgcgggggat cctcatttac ccggagacag ggag                                    34

## Description

### FIELD OF THE INVENTION

The present invention relates to a specific inhibitor of human platelet glycoprotein VI. The inhibitor may be an optionally humanized antibody or function-conservative fragments or variants thereof, a specific fusion protein or conjugate. Moreover, the present invention relates to pharmaceutical compositions containing the specific inhibitor of the invention. The present invention also relates to hybridomas expressing the antibody of the present invention. Furthermore, the present invention relates to the use of the specific inhibitor of glycoprotein VI for the preparation of a medicament for the prevention of acute and chronic vascular diseases associated with intraarterial and/or intravenous thrombosis, such as acute coronary syndrome, acute carotid artery syndrome, acute myocardial infarction, acute cerebral stroke and chronic coronary or peripheral vessel disease. Finally, the present invention relates to a process for preparing the antibody of the invention and to a process for preparing a specific fragment of the antibody of the present invention.

### BACKGROUND OF THE INVENTION

WO 01/16321 and WO 01/00810 and WO 00/68377 disclose a DNA and protein sequence of the human GPVI receptor. WO 03/05020 discloses specific binding members directed against the human glycoprotein VI (GPVI) and specific inhibitors of collagen-induced platelet aggregation. Antibodies of the single chain format and in particular single chain antibodies with a particular sequence are also disclosed. EP 1224942 and EP 1228768 disclose a monoclonal anti-GPVI antibody JAQ1, which specifically binds to mouse GPVI, for the treatment of thrombotic disease. JAQ1 antibody induces irreversible internalization of the GPVI receptor on mouse platelets. This mechanism has only been observed in mice and cannot be used in a patient. EP 1224942 does not disclose the humanized form of JAQ1 and does not provide data in man or human platelets. WO 03/008454 and WO 01/00810 disclose polypeptides, proteins and fusion proteins of GPVI as a pharmaceutical composition. Moreover, antibodies and single chains against GPVI are suggested.

Acute coronary or carotid syndromes are a major cause of death in Western societies. Even in case of an initial survival of such a cardiovascular event, many patients suffer from life-threatening complications such as intravascular thrombosis leading to further myocardial infarction or stroke.

The disruption of the atherosclerotic plaque initiates a cascade of events culminating in arterial thrombosis and ischemia of the downstream tissue, precipitating diseases such as myocardial infarction or ischemic stroke. The first response to vascular injury is adhesion of circulating platelets to exposed subendothelial matrix proteins, which triggers subsequent platelet aggregation. Among the macromolecular components of the subendothelial layer, fibrillar collagen is considered the most thrombogenic constituent, as it acts as a strong activator of platelets and supports platelet adhesion both *in vitro* and *in vivo* (Baumgartner, H. R. (1977) Platelet interaction with collagen fibrils in flowing blood. I. Reaction of human platelets with alpha chymotrypsin-digested subendothelium. *Thromb Haemost* 37, 1-16; Clemetson, K. J., Clemetson, J. M. (2001) Platelet collagen receptors. *Thromb.Haemost.* 86, 189-197; Massberg, S., Gawaz, M., Grüner, S., Schulte, V., Konrad, I., Zohlnhöfer, D., Heinzmann, U., Nieswandt, B. (2003) A crucial role of glycoprotein VI for platelet recruitment to the injured arterial wall in vivo. *J.Exp.Med.* 197, 41-49).

The platelet membrane proteins, which have been reported to be putative collagen receptors, may be divided into those which interact indirectly with collagen through collagen-bound von Willebrand factor (vWf), including GPlba and the integrin a_{IIb}b₃, and those which interact directly with collagen including GPVI, the integrin a₂b₁, and CD36 (reviewed in Clemetson, K. J., Clemetson, J. M. (2001) Platelet collagen receptors. *Thromb.Haemost.* 86, 189-197). Only recently, the platelet glycoprotein VI (GPVI) has been identified as the major platelet collagen receptor (Moroi, M., Jung, S. M., Okuma, M., Shinmyozu, K. (1989) A patient with platelets deficient in glycoprotein VI that lack both collagen-induced aggregation and adhesion. J *Clin.Invest* 84, 1440-1445). GPVI is a 60-65 kDa type I transmembrane glycoprotein, which belongs to the immunoglobulin superfamily (Clemetson, J. M., Polgar, J., Magnenat, E., Wells, T. N., Clemetson, K. J. (1999) The platelet collagen receptor glycoprotein VI is a member of the immunoglobulin superfamily closely related to FcalphaR and the natural killer receptors. J *Biol.Chem.* 274,29019-29024; Jandrot-Perrus, M., Busfield, S., Lagrue, A. H., Xiong, X., Debili, N., Chickering, T., Le Couedic, J. P., Goodearl, A., Dussault, B., Fraser, C., Vainchenker, W., Villeval, J. L. (2000) Cloning, characterization, and functional studies of human and mouse glycoprotein VI: a platelet-specific collagen receptor from the immunoglobulin superfamily. *Blood 96,* 1798-1807). In human and mouse platelets GPVI forms a complex with the FcR g-chain at the cell surface (Gibbins, J. M., Okuma, M., Farndale, R., Barnes, M., Watson, S. P. (1997) Glycoprotein VI is the collagen receptor in platelets which underlies tyrosine phosphorylation of the Fc receptor gamma-chain. *FEBS Lett.* 413, 255-259; Zheng, Y. M., Liu, C., Chen, H., Locke, D., Ryan, J. C., Kahn, M. L. (2001) Expression of the platelet receptor GPVI confers signaling via the Fc receptor gamma -chain in response to the snake venom convulxin but not to collagen. *J Biol.Chem.* 276, 12999-13006). Ligand binding to GPVI triggers tyrosine phosphorylation of the ITAM motif of the Fc receptor g chain initiating downstream signaling via Syk kinases, LAT, SLP-76, and phospholipase C (Suzuki-Inoue, K., Tulasne, D., Shen, Y., Bori-Sanz, T., Inoue, O., Jung, S. M., Moroi, M., Andrews, R. K., Berndt, M. C., Watson, S. P. (2002) Association of Fyn and Lyn with the proline rich domain of GPVI regulates intracellular signalling. *J Biol.Chem.;* Barnes, M. J., Knight, C. G., Farndale, R. W. (1998) The collagen-platelet interaction. *Curr.Opin.Hematol.* 5, 314-320; Falet, H., Barkalow, K. L., Pivniouk, V. I., Barnes, M. J., Geha, R. S., Hartwig, J. H. (2000) Roles of SLP-76, phosphoinositide 3-kinase, and gelsolin in the platelet shape changes initiated by the collagen receptor GPVI/FcR gamma-chain complex. *Blood* 96, 3786-3792; Pasquet, J. M., Gross, B., Quek, L., Asazuma, N., Zhang, W., Sommers, C. L., Schweighoffer, E., Tybulewicz, V., Judd, B., Lee, J. R., Koretzky, G., Love, P. E., Samelson, L. E., Watson, S. P. (1999) LAT is required for tyrosine phosphorylation of phospholipase cgamma2 and platelet activation by the collagen receptor GPVI. *Mol*. *Cell Biol.* 19, 8326-8334; Berlanga, O., Tulasne, D., Bori, T., Snell, D. C., Miura, Y., Jung, S., Moroi, M., Frampton, J., Watson, S. P. (2002) The Fc receptor gamma-chain is necessary and sufficient to initiate signalling through glycoprotein VI in transfected cells by the snake C- type lectin, convulxin. *Eur.J.Biochem.* 269, 2951-2960). Platelets deficient in GPVI show loss of collagen-induced adhesion and aggregation *in vitro* (Sugiyama, T., Okuma, M., Ushikubi, F., Sensaki, S., Kanaji, K., Uchino, H. (1987) A novel platelet aggregating factor found in a patient with defective collagen-induced platelet aggregation and autoimmune thrombocytopenia. *Blood* 69*,* 1712-1720). Likewise, function blocking anti-GPVI monoclonal antibodies attenuate *ex vivo* platelet aggregation in response to collagen and collagen-related peptide CRP, which mimics collagen triple helix (Sugiyama, T., Ishibashi, T., Okuma, M. (1993) Functional role of the antigen recognized by an antiplatelet antibody specific for a putative collagen receptor in platelet-collagen interaction. *Int.J.Hematol.* 58, 99-104; Schulte, V., Snell, D., Bergmeier, W., Zirngibl, H., Watson, S. P., Nieswandt, B. (2001) Evidence for two distinct epitopes within collagen for activation of murine platelets. *J Biol.Chem.* 276, 364-368). Only recently we have provided first direct *in vivo* evidence indicating that GPVI is in fact strictly required in the process of platelet recruitment under physiological shear stress following vascular injury. In different mouse models of endothelial denudation both inhibition or absence of GPVI virtually abolished platelet-vessel wall interactions and platelet aggregation, identifying GPVI as the major determinant of arterial thrombus formation (Massberg, S., Gawaz, M., Grüner, S., Schulte, V., Konrad, I., Zohlnhöfer, D., Heinzmann, U., Nieswandt, B. (2003) A crucial role of glycoprotein VI for platelet recruitment to the injured arterial wall in vivo. *J.Exp.Med.* 197,41-49).

It is known that the problem of arterial thrombus formation can be addressed by administering inhibitors of platelet aggregation. For the treatment of acute coronary syndromes, GP IIb/IIIa inhibitors such as ReoPro significantly improve the outcome of patients. However, a recent meta-analysis of clinical trials revealed a significant remaining risk for death or myocardial infarction despite optimal antithrombotic intervention (Boersma E, Harrington RA, Moliterno DJ, White H, Theroux P, Van de Werf F, de Torbal A, Armstrong PW, Wallentin LC, Wilcox RG, Simes J, Califf RM, Topol EJ, Simoons ML. Platelet glycoprotein IIb/IIIa inhibitors in acute coronary syndromes: a meta-analysis of all major randomized clinical trials. Lancet 2002; 359:189-98). Specific severe side effects of this therapeutic regimen are bleeding complications. These occurred in 2.4 % of the patients with the most severe form of intra cranial bleeding occurring in almost 0.1 % of the treated patients.

Accordingly, not only the undesired thrombosis formation is influenced, but also the general ability of the platelets to terminate bleeding. Therefore, the administration of inhibitors of platelet aggregation inherently leads to severe side effects such as bleedings, which may cause further life-threatening complications.

Antibodies directed against GPVI have been reported to induce platelet activation (Schulte, V., Snell, D., Bergmeier, W., Zirngibl, H., Watson, S. P., Nieswandt, B. (2001) Evidence for two distinct epitopes within collagen for activation of murine platelets. *J Biol.Chem.* 276, 364-368) and immuno-thrombocytopenia, hampering their use in the clinical setting.

Several mechanistic shortcomings of the GP IIb/IIIa receptor blockade have been revealed which account for suboptimal effectivity and side effects. (Dickfeld T, Ruf A, Pogatsa-Murray G, Muller I, Engelmann B, Taubitz W, Fischer J, Meier O, Gawaz M. Differential anti-platelet effects of various glycoprotein Ilb-Illa antagonists. Thromb Res. 2001;101:53-64. Gawaz M, Neumann FJ, Schomig A. Evaluation of platelet membrane glycoproteins in coronary artery disease: consequences for diagnosis and therapy. Circulation. 1999;99:E1-E11 ). Besides their ability to aggregate, platelets play a crucial role for the induction of atherosclerosis (Ruggeri ZM. Platelets in atherothrombosis. Nature Medicine 2002; 8: 1227-1234). The interaction of platelets with the endothelium via secretion of a wide variety of different vaso-active and pro-inflammatory substances from intracellular storage vesicles is of prominent importance (Massberg S, Brand K, Grüner S, Page S, Müller E, Müller I, Bergmeier W, Richter T, Lorenz M, Konrad I, Nieswandt B, Gawaz M. A Critical Role of Platelet Adhesion in the Initiation of Atherosclerotic Lesion Formation. J. Exp. Med. 2002, 196, Number 7: 887-896). Moreover GPIIb/IIIa antagonists have no influence on the release mechanism of platelet or even enhance pro-inflammatory responses such CD 40L or P-Selectin expression (for review see Bhatt DL and Topol EJ. Scientific and therapeutic advances in antiplatelet therapy. Nature Reviews Drug Discovery 2003; 2: 15-28).

Therefore, it is the problem of the invention to provide an active agent useful for avoiding life-threatening complications subsequent to an acute coronary or carotid syndrome while maintaining the potency of the blood for hemostasis. Moreover, it is the problem of the invention to provide an active agent useful for preventing or treating chronic atherosclerotic disease. Moreover, it is the problem of the invention to provide an inhibitor of glycoprotein VI, notably human glycoprotein VI, which does not activate the GPVI receptor by intrinsic antibody activity and which does not induce immuno-thrombocytopenia. Moreover, it is the problem of the invention to provide an inhibitor for the release mechanism of platelets and the expression of pro-inflammatory responses.

The present invention is directed to a purely inhibitory anti-GPVI antibody or function-conservative fragments or variants thereof for the treatment of acute vascular diseases. Moreover, the invention addresses the problem of providing a pharmaceutical composition for the treatment of acute vascular complications such as intra-vascular thrombosis especially in patients, with atherosclerosis and/or acute endothelial lesions. Moreover, the present invention addresses the problem of providing a pharmaceutical composition for the treatment of chronic coronary artery and peripheral vessel disease. The present invention provides a pharmaceutical composition for the treatment of acute coronary, carotid artery and peripheral vessel diseases as well as chronic atherosclerosis, wherein the medicament contains a specific optionally humanized antibody hGP 5C4, or a fragment thereof, notably an Fab fragment, a function-conservative variant thereof, a fusion protein or conjugate thereof.

### SUMMARY OF THE INVENTION

The above problems are solved according to the claims. In a first aspect, the present invention provides monoclonal antibody hGP 5C4 or function-conservative fragments, notably hGP 5C4 Fab, or variants thereof which specifically bind to glycoprotein VI, in particular to human glycoprotein VI. The monoclonal antibody may be purified from an antibody producing cell designated hGP 5C4 deposited with *DSMZ-Deutsche Sammlung von Mikroorganismen und Zellkulturen* under Accession No. 2631, or progeny thereof. The monoclonal antibody or function-conservative fragments or variants thereof preferably inhibit collagen-binding of human glycoprotein VI. The fragment is preferably a Fab fragment obtainable by papain digestion of hGP 5C4 antibody. The present invention also provides humanized antibodies specifically binding to glycoprotein VI. Moreover, the present invention provides fusion proteins specifically binding to glycoprotein VI, which comprise an amino acid sequence of hGP 5C4 or function-conservative fragments thereof, notably hGP 5C4 Fab, or variants thereof. Moreover, the present invention also provides conjugates containing an effector moiety and the monoclonal antibody hGP 5C4 or function-conservative fragments, notably hGP 5C4 Fab, or variants thereof. The inhibitor of the present invention preferably does not activate platelets or does not induce immuno-thrombocytopenia. Moreover, the inhibitor of the present invention preferably inhibits the release mechanism of platelets and the expression of pro-inflammatory responses.

A second aspect of the present invention relates to a hybridoma cell line deposited as hGP 5C4 with DSMZ-Deutsche Sammlung von Mikroorganismen und Zellkulturen on November 25, 2003 under accession number 2631, or progeny thereof.

A third aspect of the present invention relates to a nucleic acid coding for monoclonal antibody hGP 5C4 or a function-conservative fragment, notably hGP 5C4 Fab, or variants thereof, notably due to the degeneracy of the genetic code, wherein said nucleic acid is obtainable from the hybridoma cell line of the present invention, or progeny thereof. The present invention also relates to a nucleic acid coding for a polypeptide comprising at least 5 consecutive amino acids of the monoclonal antibody hGP 5C4 and binding specifically to human glycoprotein VI.

A fourth aspect of the present invention relates to a pharmaceutical composition comprising a pharmaceutically acceptable excipient and the inhibitor of the present invention.

A sixth aspect of the present invention relates to the use of the inhibitor of the present invention for the preparation of a medicament for the prevention or treatment of acute or chronic vascular diseases associated with intraarterial or intravenous thrombosis. Preferably, the medicament is administered parenterally.

A seventh aspect of the invention relates to a process for producing monoclonal antibody hGP 5C4 or function-conservative fragments thereof, said process comprising
(i) culturing hybridoma hGP 5C4, or progeny thereof in medium under conditions conducive to expression of antibody therefrom,
(ii) obtaining antibody hGP 5C4 from the culture medium; and
(iii) optionally preparing a Fab fragment of antibody hGP 5C4 by enzymatic digestion. The present invention also relates to a process for preparing hGP 5C4 Fab, said process comprising the step of digesting hGP 5C4 antibodies and isolating the Fab fragments. Preferably, hGP 5C4 is digested by using papain or a derivative thereof.

### DESCRIPTION OF THE FIGURES

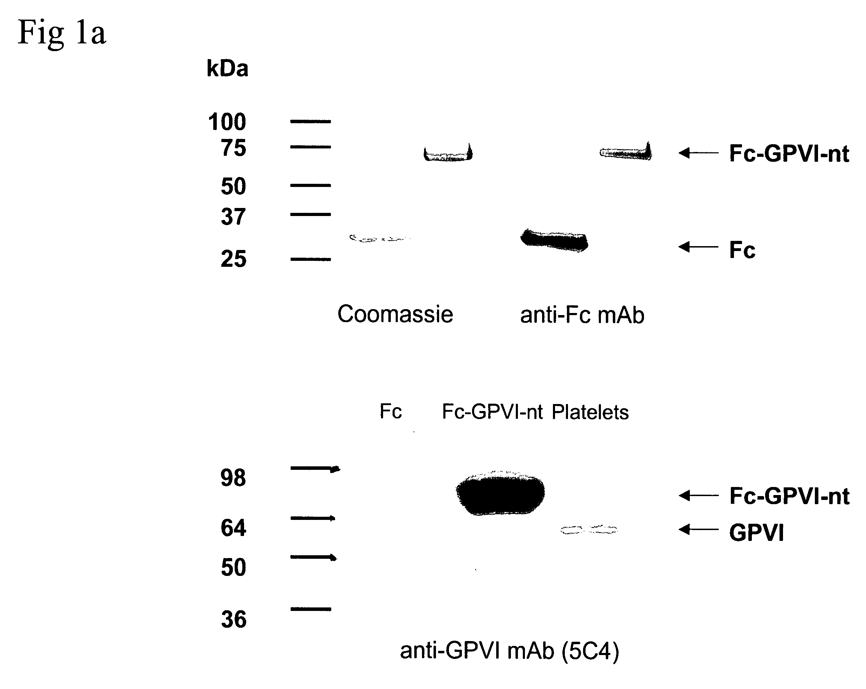

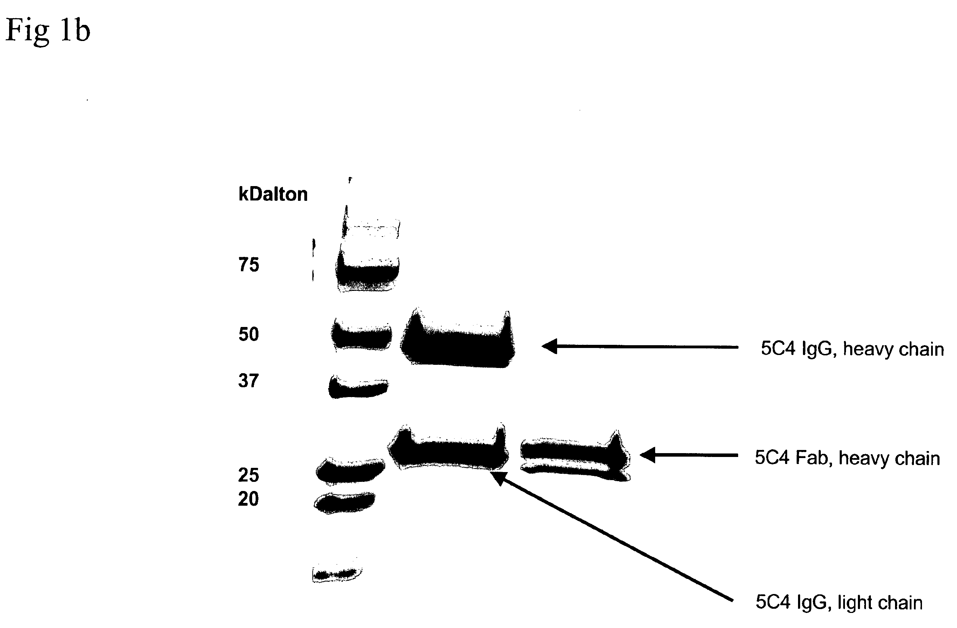

**Figure 1** *Characterization of antigen binding of hGP 5C4*. (**a**) *upper panel:* Fc-GPVI-nt and control Fc lacking the extracellular GPVI domain were applied for SDS-PAGE under reducing conditions. Coomassie blue stain (left) and immunoblotting with peroxidase-conjugated goat anti-human Fc antibody (right) identified Fc-GPVI-nt with a molecular mass of ∼80kDa. *Lower panel*: Immunoblotting of Fc, Fc-GPVI-nt, or human platelets using the anti-GPVI monoclonal antibody hGP 5C4. hGP 5C4 specifically detected purified Fc-GPVI-nt fusion protein and GPVI receptor on platelets, but not the control Fc protein. (b) The generation of Fab fragments of the IgG hGP 5C4 was verified in a SDS gel with Coomassie staining after digestion with an ImmunoPure Fab Kit (Pierce Biotechnology, Inc., Rockford, IL, USA).

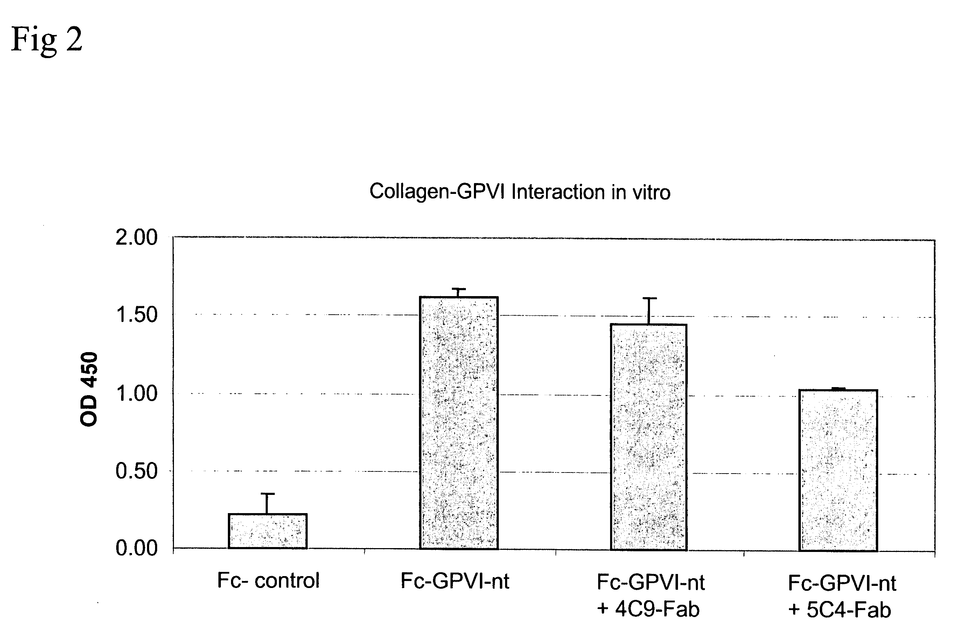

**Figure 2** *The ability of hGP 5C4 Fab to inhibit GPVI binding to collagen was monitored in an ELISA-* based assay. Adhesion of the Fc-GPVI-nt - consisting of the extracellular domain of GPVI and the Fc part of an IgG - to immobilised collagen was investigated in the presence of different anti-GPVI antibody Fab fragments (20 µg/ml). Binding of the Fc part without the GPVI receptor domain served as control. The binding is visualised with a secondary antibody directed to the Fc part of Fc-GPVI-nt labelled with peroxidase. Peroxidase (PE) is finally detected by an ELISA system measuring the absorption photometrically at 450 nm. hGP 5C4 prevents Fc-GPVI-nt binding to collagen, whereas 4C9 could not prevent collagen interaction with GPVI. The means ± SEM are shown.

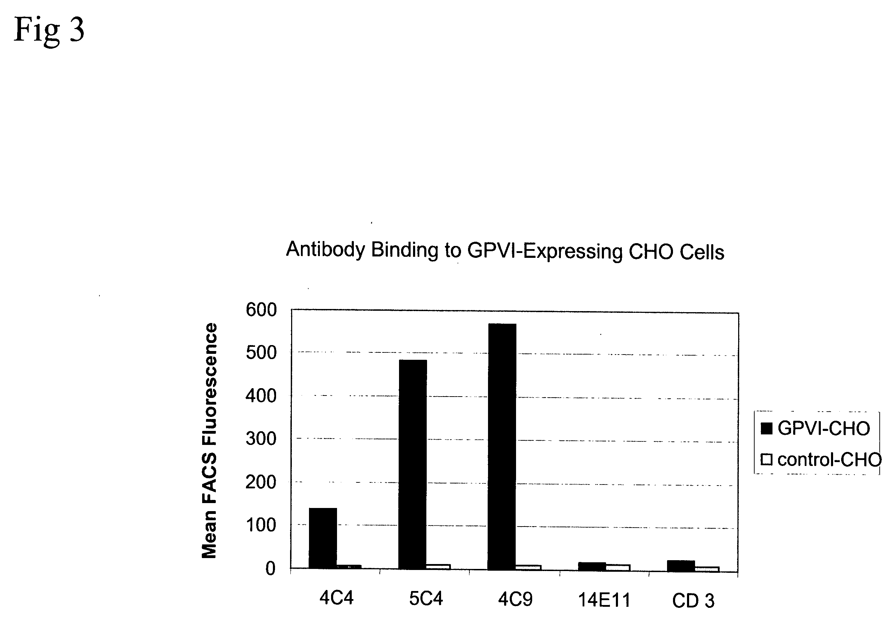

**Figure 3** *The binding of different antibodies to stable GPVI-expressing CHO cells was measured by FACS.* The binding of the specific antibodies to control CHO cells served as control. After incubation with the primary antibodies CHO cells were incubated with anti-IgG rat antibodies labelled with PE. Specific PE fluorescence was determined in a Becton Dickenson FACScalibur device.

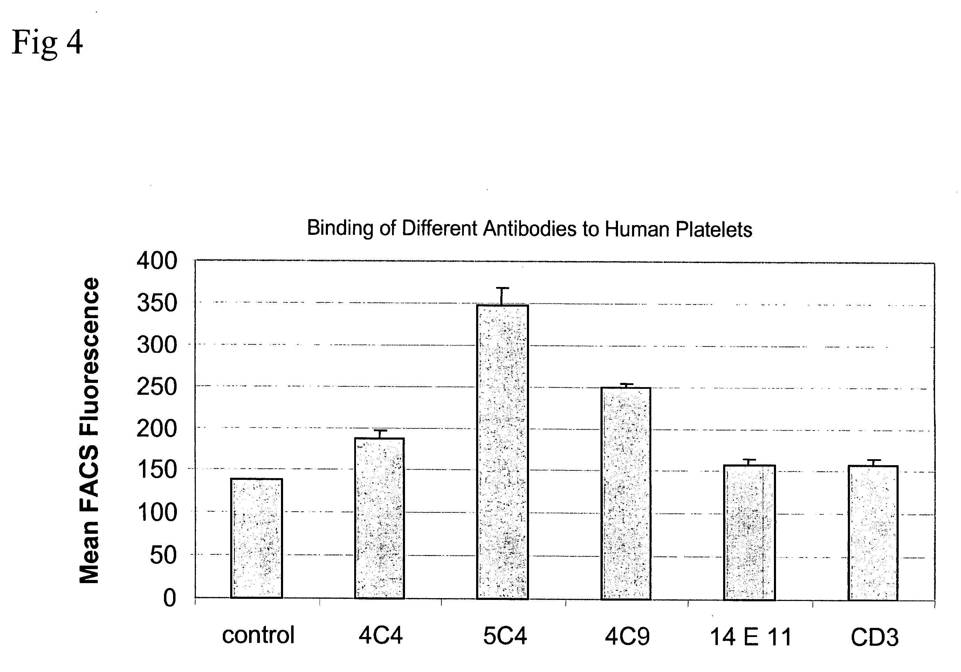

**Figure 4** *The binding of different antibodies to human platelets was determined by FACS.* Platelets were washed and platelet rich plasma was prepared as described in Material and Methods. After incubation with the primary antibodies, human platelets were incubated with anti-IgG rat antibodies labeled with peroxidase (PE). Specific PE fluorescence was determined in a Becton Dickenson FACScalibur device. The means ± SEM are shown.

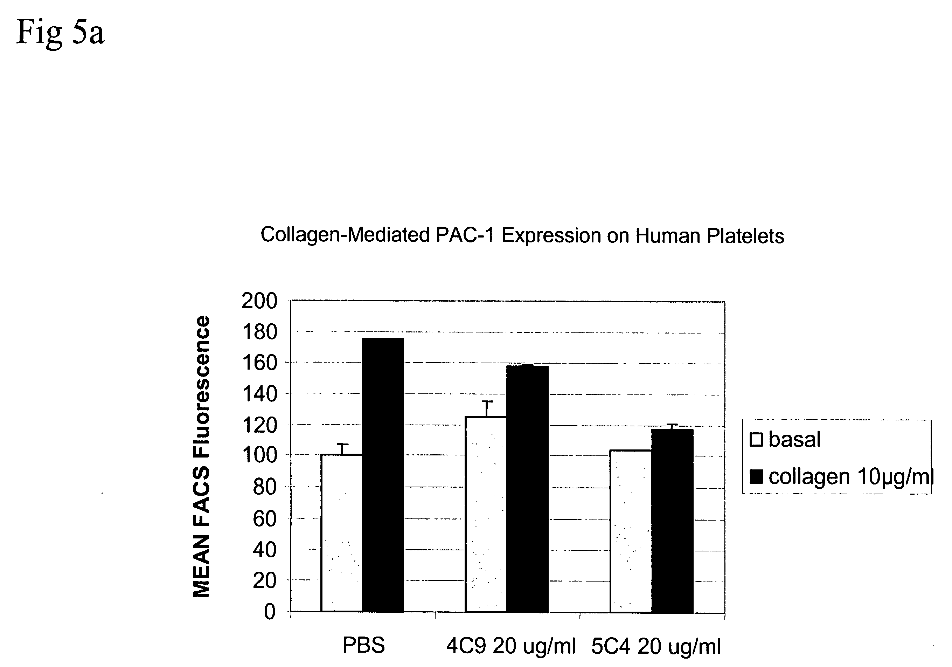

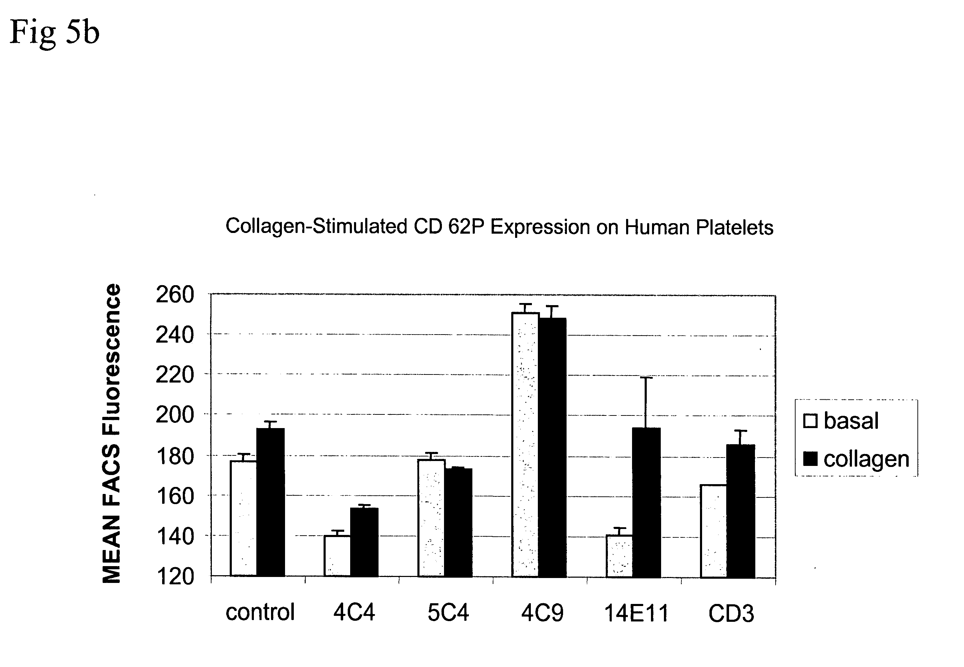

**Figure 5** *The ability of different anti-GPVI antibodies to inhibit collagen-mediated platelet activation was measured for different activation markers by FACS.* **(a)** Incubation with bovine collagen type I (10 µg/ml) activated PAC-1 on human platelets. Preincubation with the anti-GPVI antibody hGP 5C4 Fab (20 µg/ml) prevented PAC-1 activation, whereas 4C9 Fab (20 µg/ml) led to additional stimulation of platelets and could not prevent collagen-mediated PAC-1 activation. Specific PAC-1 fluorescence was determined in a Becton Dickenson FACScalibur device. The means ± SEM are shown. **(b)** Pre-incubation of human platelets with hGP 5C4 Fab (20 µg/ml) inhibited CD 62 P activation by collagen type 1 (10 µg/ml), whereas other antibodies could not prevent collagen-mediated CD 62P activation. 4C9 (20 mg/ml) or 14E11 (20 µg/ml) had even stimulating effects on human platelets. The means ± SEM are shown.

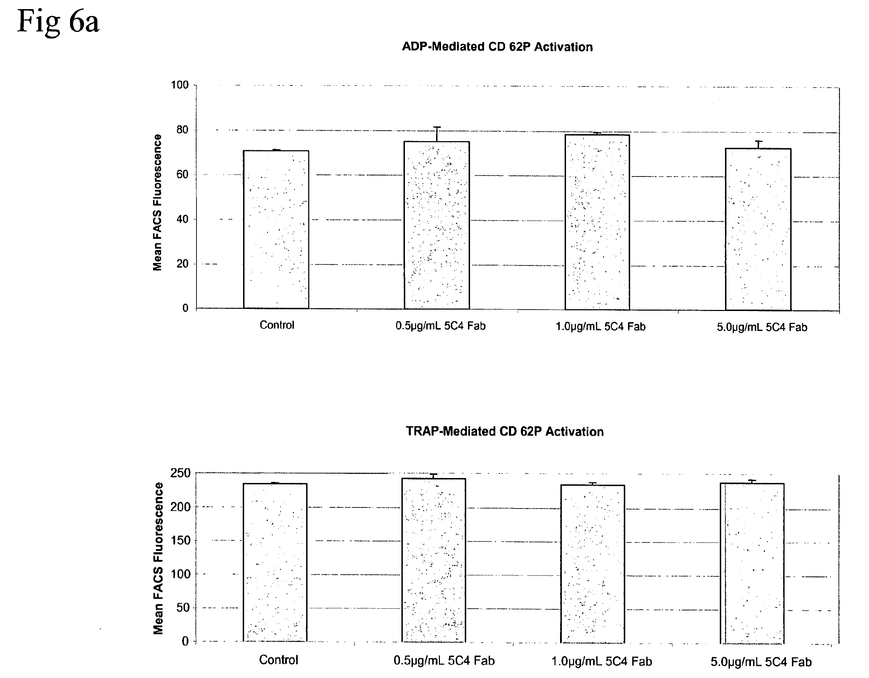

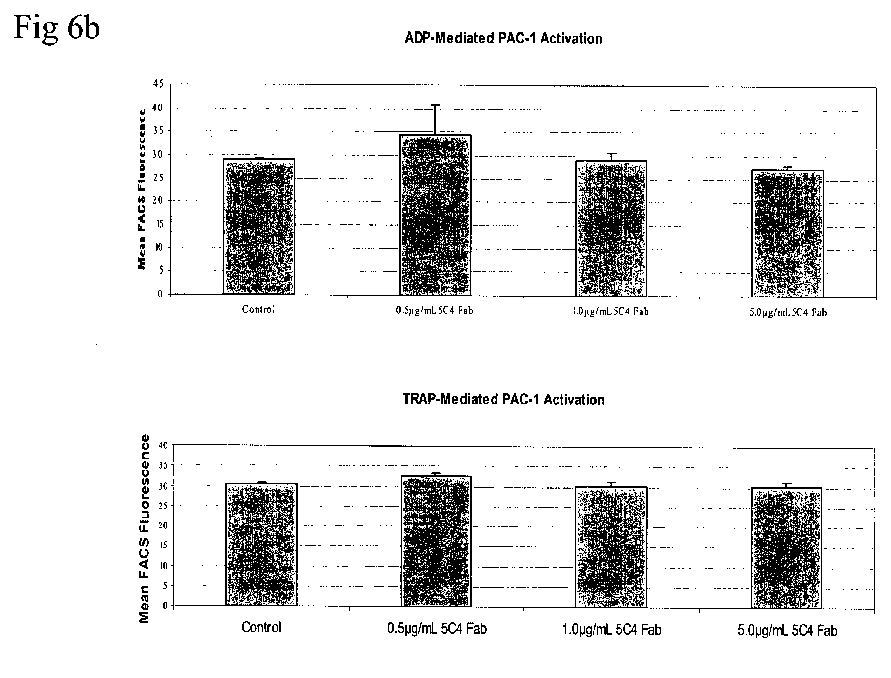

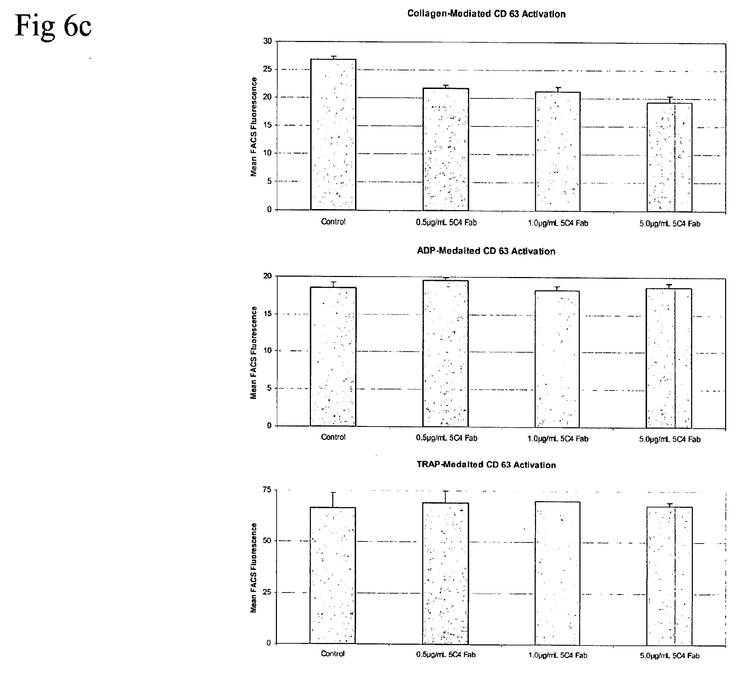

**Figure 6** *The specificity of hGP 5C4 for collagen-mediated processes was investigated by ADP- and thrombin induced human platelet activation in FACS.* **(a)** Preincubation with 0.5 µg/ml to 5 µg/ml hGP 5C4 Fab with human platelets was followed by ADP (20 µM) stimulation (top). The CD 62 P expression was determined in a Becton Dickenson FACScalibur device with specific antibodies as described in Material and Methods. hGP 5C4 had no influence on ADP-mediated CD 62 P activation in human platelets. The TRAP-mediated activation of CD62P was also tested (bottom). hGP 5C4 had no effect on TRAP (25µM)-mediated CD62P activation in human platelets. The means ± SEM are summarized. **(b)** Human platelets were incubated with 0.5 µg/ml to 5 µg/ml hGP 5C4 Fab and stimulated with ADP (20 µM; top) or TRAP (25µM; bottom). PAC-1 fluorescence was measured in a Becton Dickenson FACScalibur. hGP 5C4 had no influence on ADP- mediated or TRAP-mediated PAC-1 activation. The means ± SEM are shown. **(c)** Collagen (10µg/ml), ADP (20µM) and TRAP-mediated (25µM) platelet activation and the effect of hGP 5C4 Fab on CD63 activation was investigated. hGP 5C4 inhibited collagen-mediated CD63 activation, whereas ADP- and TRAP- mediated CD63 activation was unaffected. The means ± SEM are ssummarized.

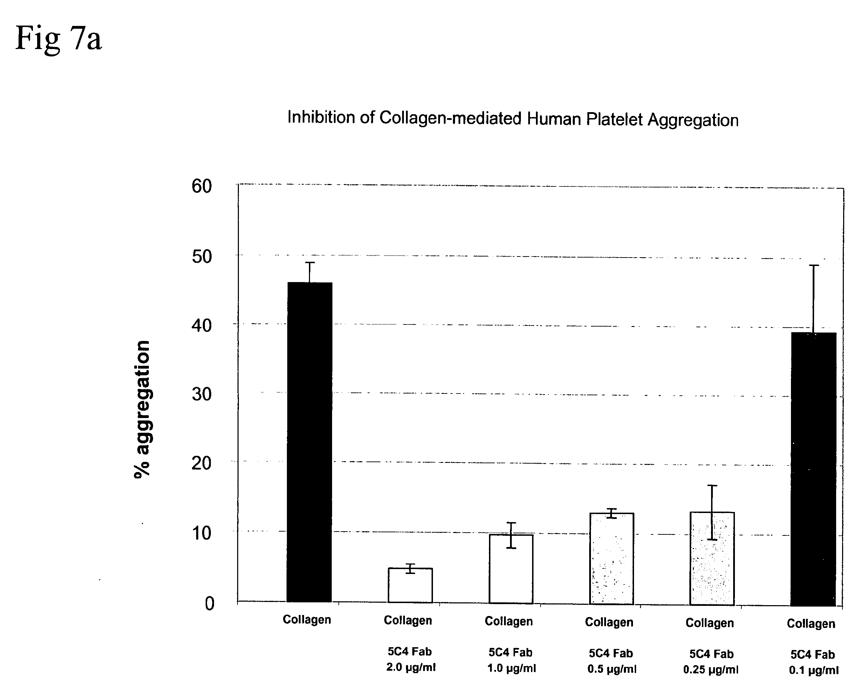

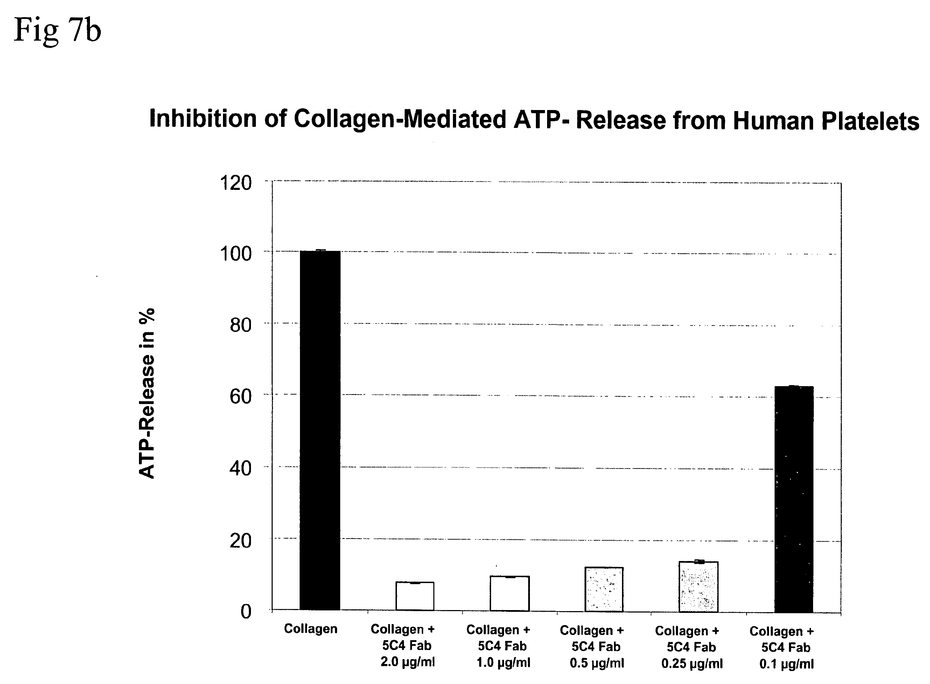

**Figure 7** *The inhibition of collagen-mediated aggregation and A TP release by hGP 5C4 was tested in human platelets.* Increasing concentrations of hGP 5C4 Fab (0.1 µg/ml to 2 µg/ml) were incubated with human platelets and collagen-induced (3 µg/ml) aggregation and ATP release was measured simultaneously ex vivo in an aggregometer. (a) The aggregation is expressed relative to an internal standard [in %] (see Material and Methods for description). From 0.25 µg/ml hGP 5C4 Fab concentration the collagen-mediated aggregation of human platelets was almost completely abolished. **(b)** In simultaneous experiments, ATP release was measured form these platelets. Collagen induced marked release of ATP from intracellular stores. hGP 5C4 potently inhibited this collagen-induced ATP release. The ATP release is given in % of control release. The means ± SEM are shown.

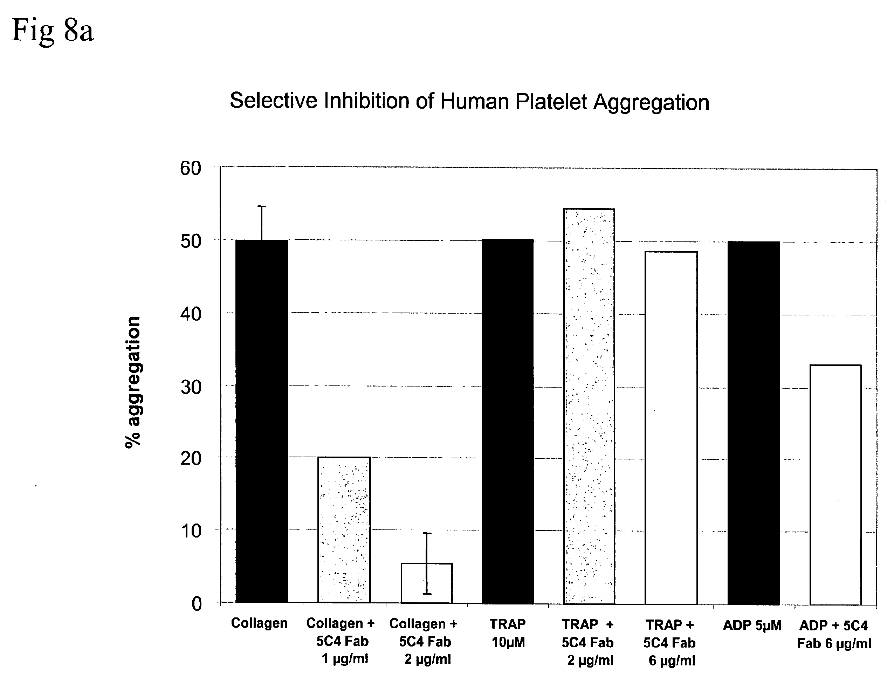

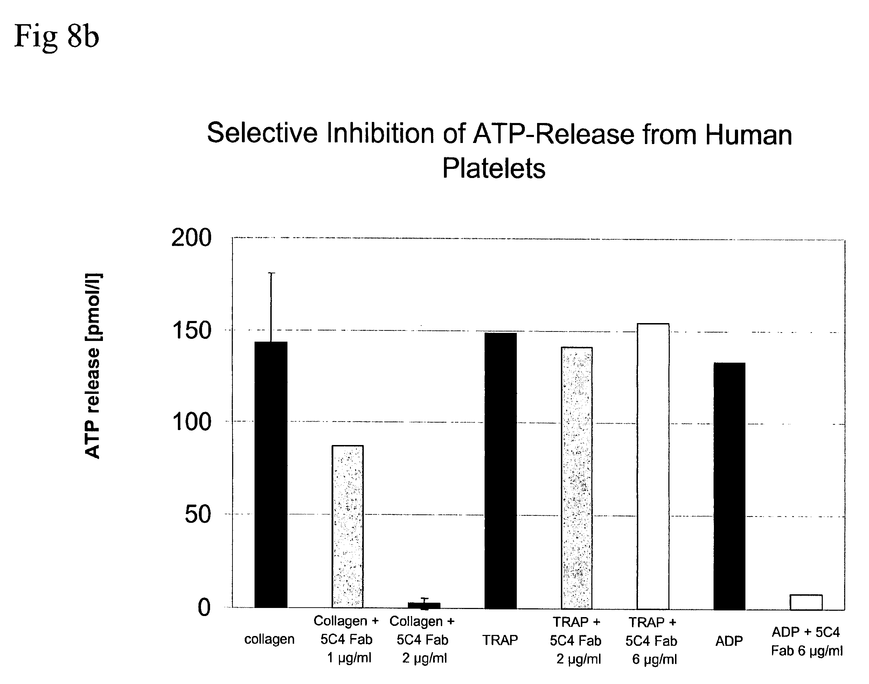

**Figure 8** *The specificity of hGP 5C4Fab for collagen-mediated inhibition of human platelet aggregation and ATP release was tested.* **(a)** Different agonists (collagen 2 µg/ml, TRAP 10 mmol/l, and ADP 5 µmol/l) were used to induce aggregation *ex vivo* in an aggregometer [in % of internal standard](see Methods for description). hGP 5C4 Fab (1 µg/ml and 2 µg/ml) almost completely abolished the collagen-mediated aggregation of human platelets. TRAP- and ADP-mediated aggregation was largely unaffected by substantially higher doses of hGP 5C4 Fab (2 µg/ml and 6 µg/ml). **(b)** ATP release was measured simultaneously given in pmol ATP/l. hGP 5C4 (1 µg/ml and 2 µg/ml) inhibited collagen-mediated ATP release. Substantially higher concentrations of hGP 5C4 Fab (2 µg/ml and 6 µg/ml) had no effect on thrombin/TRAP-mediated ATP release. The highest dose of hGP 5C4 Fab (6 µg/ml) also inhibited ADP-mediated ATP release. The means ± SEM are shown.

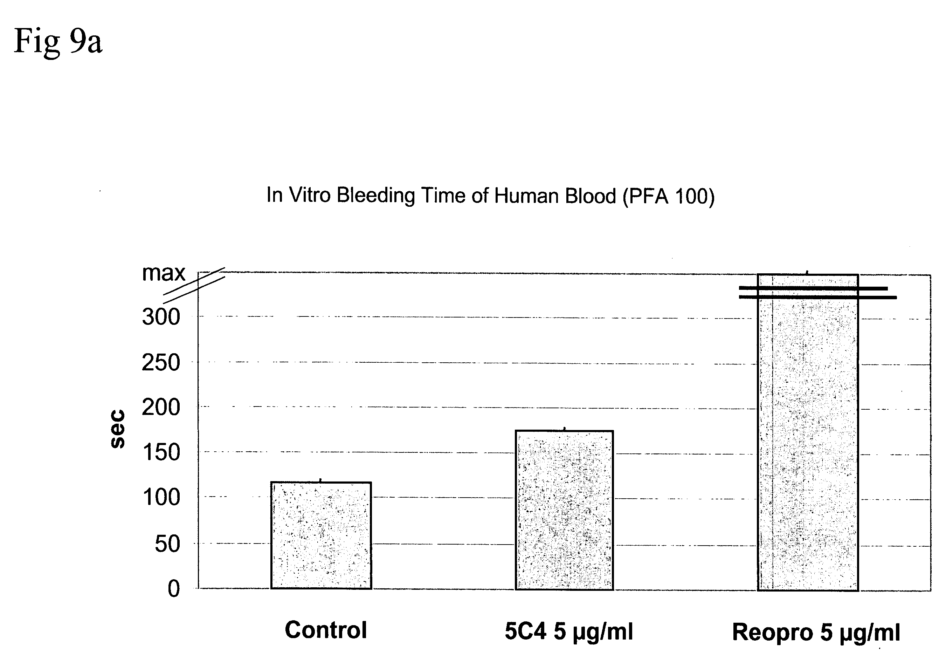

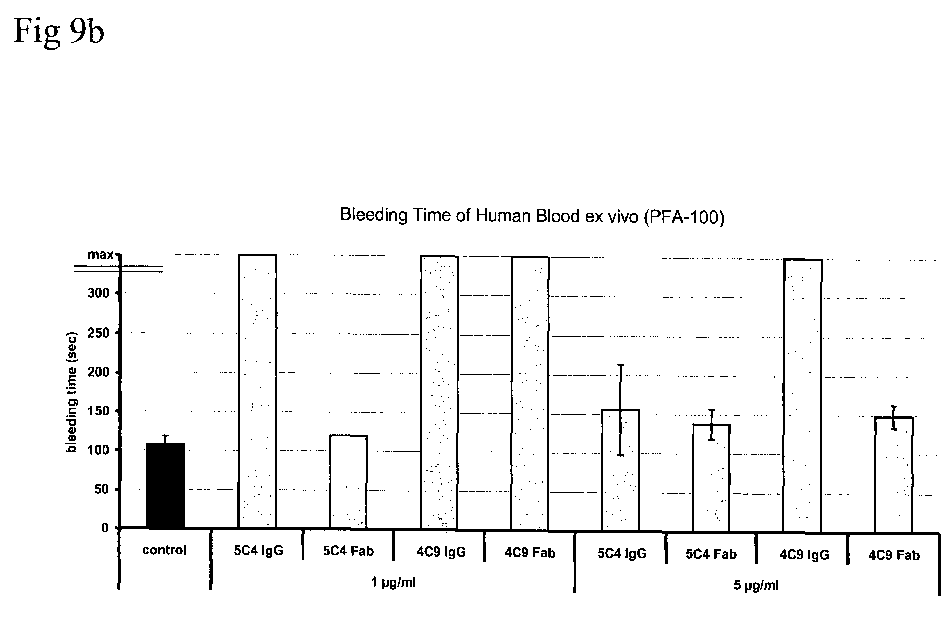

**Figure 9** *The influence of hGP 5C4 on bleeding time was tested in human blood ex vivo.* **(a)** Human blood was incubated with hGP 5C4 Fab in 10 to 20 fold therapeutic concentrations (5 µg/ml) and bleeding time was determined with a PFA-100 device (see Methods for description). Bleeding time was compared to ReoPro^{R} in equivalent concentrations. There was no significant prolongation of the bleeding time with hGP 5C4 Fab compared to control blood, whereas Reopro led to a maximal prolongation of bleeding time. (b) hGP 5C4 and 4C9 in different antibody formats were tested for bleeding time of human blood. hGP 5C4 Fab (1 µg/ml and 5 µg/ml) did not show any prolongation of bleeding time. In contrast, 4C9 markedly prolonged bleeding time both as Fab and as IgG antibody. The means ± SEM are shown.

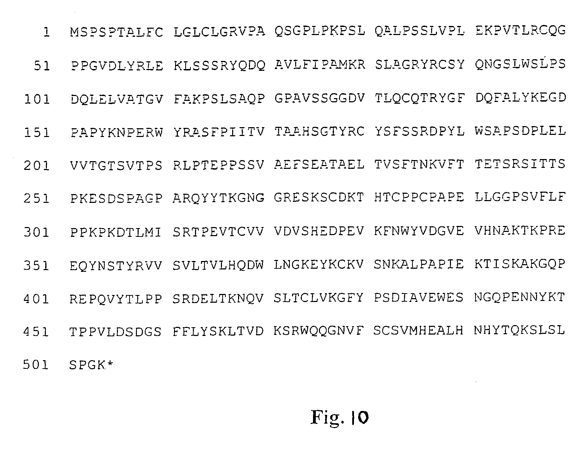

**Figure 10** Amino acid sequence of Fc-GPVI-nt used as a dimer for the preparation of the antibody of the present invention.

### DETAILED DESCRIPTION OF THE INVENTION

### Definitions

"hGP 5C4" is a specific antibody raised against human glycoprotein VI. The generation and characterization of hGP 5C4 is described in the Examples. A hybridoma expressing hGP 5C4 has been deposited with deposited with *DSMZ-Deutsche Sammlung von Mikroorganismen und Zellkulturen* under Accession No. 2631. "hGP 5C4 Fab" is the Fab fragment of hGP 5C4. The generation and characterization of hGP 5C4 Fab is described in the examples.

"Immunological activity" of hGP 5C4 and hGP 5C4 Fab refers to any of the following activities: ability to specifically bind human glycoprotein VI; ability to inhibit the binding of human glycoprotein VI to collagen in a specific manner; lack of activation of platelets or induction of immuno-thrombocytopenia; inhibition of the release mechanism of platelets and the expression of pro-inflammatory responses.

hGP 5C4 or hGP 5C4 Fab "activity" or "function" refers to any of the immunological activities of hGP 5C4 or hGP 5C4 Fab, or to any other biological activity ascribed to hGP 5C4 or hGP 5C4 Fab in this disclosure, including the role of hGP 5C4 or hGP 5C4 Fab in the prevention or treatment of acute or chronic cardiovascular disease associated with intraarterial and/or intravenous thrombosis.

The "variable region" of hGP 5C4 refers to the variable region of the hGP 5C4 light chain or the variable region of the hGP 5C4 heavy chain.

A "nucleic acid" is a polymeric form of nucleotides of any length, which contain deoxyribonucleotides, ribonucleotides, and analogs in any combination. Nucleic acids may have any three-dimensional structure, and may perform any function, known or unknown. The term "nucleic acid" includes double-, single-stranded, and triple-helical molecules. Unless otherwise specified or required, any embodiment of the invention described herein that is a nucleic acid encompasses both the double-stranded form and each of two complementary single-stranded forms known or predicted to make up the double stranded form.

The term "recombinant" polynucleotide as used here intends a polynucleotide of genomic, cDNA, semisynthetic, or synthetic origin which either does not occur in nature or is linked to another polynucleotide in an arrangement not found in nature.

The terms "polypeptide", "peptide" and "protein" are used interchangeably herein to refer to polymers of amino acids of any length. The polymer may be linear or branched, it may comprise modified amino acids or amino acid analogs, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labelling component.

The term "function conservative fragments or variants of hGP 5C4" includes any peptide, polypeptide or protein monomer or polymer with hGP 5C4 Fab activity, derived from hGP 5C4 IgG antibody, and smaller and larger functionally equivalent polypeptides, as described herein. A "function conservative fragment or variant" of a hGP 5C4 polypeptide or polynucleotide varies from the native sequence by any combination of additions deletions, or substitutions while preserving at least the affinity to human glycoprotein VI. A function-conservative fragment may be obtained by digesting hGP 5C4 with a suitable enzyme or by chemical synthesis of a portion of hGP 5C4. A typical fragment is hGP 5C4 Fab. A function-conservative fragment of a hGP 5C4 polynucleotide either encodes a polypeptide that is functionally equivalent to hGP 5C4 Fab when used in an expression system, or has similar hybridization specificity as a hGP 5C4 Fab polynucleotide when used in a hybridization assay.

A "humanized antibody molecule" as used in this specification is a molecule containing an antigen binding site derived from hGP 5C4, and remaining immunoglobulin-derived parts of the molecule being derived from a human immunoglobulin. The antigen binding site typically comprises complementarity determining regions (CDRs) of hGP 5C4 which determine the binding specificity of the hGP 5C4 antibody and which are carried on appropriate framework regions in the variable domains. The term "humanized" in relation to hGP 5C4 antibodies as used in the present specification includes any method of humanization such as for example CDR grafting or chimeric antibody preparation or any hybrid thereof such as for example a CDR grafted heavy chain in combination with a chimerized light chain.

A "cell line" or "cell culture" denotes higher eukaryotic cells gown or maintained in vitro. It is understood that the progeny of a cell may not be completely identical (either morphologically, genotypically, or phenotypically) to the parent cell.

"Heterologous" means derived from a genotypically distinct entity from the rest of the entity to which it is being compared. For example, a polynucleotide may be placed by genetic engineering techniques into a plasmid or vector derived from a different source, and is a heterologous polynucleotide. A promoter removed from its native coding sequence and operatively linked to a coding sequence with which it is not naturally found linked is a heterologous promoter.

An "isolated" polynucleotide or polypeptide is one that is substantially free of the materials with which it is associated in nature. By substantially free is meant at least 50%, preferably at least 70%, more preferably at least 80%, and even more preferably at least 90% free of the materials with which it is associated in nature.

As used herein, "treatment" refers to clinical intervention in an attempt to alter the natural course of the individual or cell being treated, and may be performed either for prophylaxis or during the course of clinical pathology. Desirable effects include preventing occurrence or recurrence of disease, alleviation of symptoms, diminishment of any direct or indirect pathological consequences of the disease, lowering the rate of disease progression, amelioration or palliation of the disease state, and remission or improved prognosis.

Following rupture of the atherosclerotic plaque, exposure of subendothelial collagen is the major trigger that initiates platelet adhesion and aggregation at the site of injury, followed by arterial thrombosis (van Zanten, G. H., de Graaf, S., Slootweg, P. J., Heijnen, H. F., Connolly, T. M., de Groot, P. G., Sixma, J. J. (1994) Increased platelet deposition on atherosclerotic coronary arteries. *J Clin.Invest* 93, 615-632; Baumgartner, H. R., Muggli, R., Tschopp, T. B., Turitto, V. T. (1976) Platelet adhesion, release and aggregation in flowing blood: effects of surface properties and platelet function. *Thromb.Haemost.* 35, 124-138). The platelet glycoprotein GPVI, which has been cloned recently (Clemetson, J. M., Polgar, J., Magnenat, E., Wells, T. N., Clemetson, K. J. (1999) The platelet collagen receptor glycoprotein VI is a member of the immunoglobulin superfamily closely related to FcalphaR and the natural killer receptors. *J Biol.Chem.* 274, 29019-29024; Jandrot-Perrus, M., Busfield, S., Lagrue, A. H., Xiong, X., Debili, N., Chickering, T., Le Couedic, J. P., Goodearl, A., Dussault, B., Fraser, C., Vainchenker, W., Villeval, J. L. (2000) Cloning, characterization, and functional studies of human and mouse glycoprotein VI: a platelet-specific collagen receptor from the immunoglobulin superfamily. Blood 96, 1798-1807), has been identified to be the major platelet collagen receptor, mediating platelet adhesion both *in vitro* (Chen, H., Locke, D., Liu, Y., Liu, C., Kahn, M. L. (2002) The platelet receptor GPVI mediates both adhesion and signaling responses to collagen in a receptor density-dependent fashion. *J Biol. Chem.* 277, 3011-3019) and under (patho-)physiological conditions *in vivo* (Massberg, S., Gawaz, M., Grüner, S., Schulte, V., Konrad, I., Zohlnhöfer, D., Heinzmann, U., Nieswandt, B. (2003) A crucial role of glycoprotein VI for platelet recruitment to the injured arterial wall in vivo. *J.Exp.Med.* 197, 41-49). This identifies the inhibition of GPVI as a promising strategy to prevent platelet recruitment and arterial thrombosis in patients with advanced atherosclerosis.

Therefore, the GPVI-endothelium interaction for platelet adhesion is the initial step of intravascular thrombosis. Thus, an effective anti-platelet drug for the prevention of collagen GPVI interactions is an ideal treatment for this initial step of platelet adhesion without undesired side effects. Therefore, the invention provides an inhibitor such as a specific antibody or a fragment thereof or a function conservative variant thereof against GPVI. The protein structure of the antibody is novel. This antibody specifically binds to GPVI as purified protein, GPVI expressed on the surface of GPVI expressing CHO cells and native GPVI on the surface of freshly isolated human platelets. Moreover, the hGP 5C4 Fab prevents collagen - GPVI interaction *in vitro* and hence can block the ligand-receptor interaction of collagen and GPVI.

The antibody fragment hGP 5C4 Fab has marked inhibitory effects on the main physiological functions of platelets induced by collagen stimulation. The stimulation of collagen-mediated physiological activation parameters PAC-1 and CD 62P-Selectin was completely prevented by hGP 5C4 Fab. Other putative anti-GPVI antibodies had no significant inhibitory effect on PAC-1 and CD 62 P. Moreover, other putative anti-GPVI antibodies presented with a well known problem for inhibitors: Despite specific binding to GPVI some antibodies like 14E11 and 4C9 activated PAC-1 and CD 62P even in the absence of agonists. This is a common problem for the development of inhibitory antibodies. The antibody fragment hGP 5C4 Fab did not show any intrinsic GPVI activity. Additionally, hGP 5C4 Fab potently inhibited human platelet aggregation *ex vivo* without any intrinsic activity.

Moreover, the inhibitory effect of hGP 5C4 was highly selective for collagen-mediated effects. The inhibitory antibody had no effects on ADP-mediated activation of PAC-1 and CD 62P. Additionally, hGP 5C4 Fab had no effect on TRAP- and ADP-mediated aggregation and ATP release of human platelets ex vivo. As a consequence, hGP 5C4 Fab is a highly selective inhibitor of arterial thrombosis with no effects on venous thrombosis and hemostasis. Our experiments clearly showed that bleeding time of human blood was not prolonged in the PFA-100 device. Thus, hGP 5C4 Fab circumvents an almost inherent problem of anti-platelet drugs (Quinn MJ; Plow EF; Topol EJ. 2002: Platelet Glycoprotein IIb/IIIa inhibitors - Recognition of a two-edged sword. Circulation 106: 379-385; Bhatt DL & Topol EJ; 2003: Scientific and therapeutic advances in antiplatelet therapy. Nat Rev Drug Discov 2: 15-28). hGP 5C4 Fab shows highly potent and selective inhibition of platelet activation with no prolongation of bleeding time. Thus, hGP 5C4 is an ideal drug for the treatment of acute vascular syndromes like acute coronary syndromes or ischemic stroke without unwanted and potentially fatal side effects like intra cranial hemorrhage or other bleeding complications. Moreover, hGP 5C4 Fab shows potent and highly selective inhibition of release of transmitter substances such as ATP from intracellular storage vesicles of human platelets. As this is a crucial parameter for the platelet-endothelium interaction promoting atherosclerosis, the antibody hGP 5C4 is drug for the treatment and prevention of atherosclerosis. Accordingly, the invention solves the problem of treatment of atherosclerosis by inhibition of platelet secretion.

The antibody hGP 5C4 of the invention is a novel GPVI inhibitor selectively inhibiting the activated branch of GPVI mediated effects without significant bleeding complications. The antibody hGP 5C4 and the antibody fragment hGP 5C4 Fab can be used for the treatment of atherosclerotic complications caused by unstable atherosclerotic plaques with plaque rupture or endothelial lesion. Therefore, the antibody hGP 5C4 and the antibody fragment hGP 5C4 Fab serve as therapeutic inhibitors for collagen-mediated GPVI activation without affecting the intrinsic activity of the GPVI receptor with the relevant signalling system. Moreover, these inhibitors can be used for the prevention and treatment of atherosclerosis.

Based on the recent improvements in imaging techniques by intravascular ultrasound or nuclear magnetic resonance imaging, it is possible to identify patients with atherosclerosis being at risk of acute clinical complications such as acute coronary or carotid syndrome, whereby the patients have active lesions as possible causes for intravascular thrombosis. It is then possible by the present invention to prevent the formation of intravascular thrombosis by the administration of a medicament containing the antibody hGP 5C4 against platelet GPVI without undesired side effects.

Active lesions are characterized by the unmasking of subendothelial matrix collagens and platelet activation. The occurrence of such lesions can be investigated e.g. by intravascular ultrasound or thermography (e.g., Fayed and Fuster, Clinical imaging of the high-risk or vulnerable atherosclerotic plaque. Circulation 2001; 89:305-316) or nuclear resonance imaging (Helft et al., Progression and Regression of Atherosclerotic Lesions. Circulation 2002; 105:993-998). Moreover, the dimeric form of the Fc-GPVI-nt fusion protein serves as and ideal diagnostic tool for the identification of endothelial lesions in patients (EP 03/05929). Such lesions are highly probable in patients with acute coronary or carotid syndromes, and the risk of the reoccurrence of acute clinical complications such as myocardial infarction or stroke is very high, decreasing progressively with increasing time distance from the primary event.

Therefore, the present invention provides a method of treating a patient suffering from an acute coronary or carotid syndrome for avoiding intravascular thrombosis. Moreover, based on the present invention, it is possible to treat patients being at risk of intravascular thrombosis due to the rupture of complex arteriosclerotic plaques. The rupture also unmasks the subendothelial collagen matrix. As a consequence of intraarterial thrombus formation, the perfusion of vital organs is blocked with the above described important and life threatening clinical syndromes.

### Products of the invention:

The present invention relates to monoclonal antibody hGP 5C4 which specifically binds to human glycoprotein VI. The present invention encompasses fragments or function-conservative variants of monoclonal antibody hGP 5C4 such as the hGP 5C4 Fab fragment or polypeptide fragments of hGP 5C4 Fab containing at least a portion of a variable region of hGP 5C4 Fab. Preferred fragments are those with immunological activity of hGP 5C4 Fab. Also preferred are fragments which comprise amino acid sequences substantially different from other immunoglobulins, and fragments comprising a complementarity defining region (CDR). In one embodiment, the invention includes a polypeptide fragment of the hGP 5C4 Fab light chain variable region, comprising at least 5 consecutive amino acids, more preferably 15 consecutive amino acids, still more preferably 30 consecutive amino acids. In other embodiments, the invention includes a polypeptide fragment of the hGP 5C4 Fab heavy chain variable region, comprising the 5 amino acids.

The size of the hGP 5C4 polypeptide fragments may be only the minimum size required to provide a desired function. It may optionally comprise additional sequence, either native to hGP 5C4, or from a heterologous source. hGP 5C4 fragments may contain only 5 consecutive amino acids from a hGP 5C4 Fab variable region sequence. Polypeptides comprising 7 amino acids, more preferably about 10 amino acids, more preferably about 15 amino acids, more preferably about 25 amino acids, more preferably about 50 amino acids, more preferably about 75 amino acids from the hGP 5C4 Fab light or heavy chain variable region are also included. Even more preferred are polypeptides comprising the entire hGP 5C4 Fab light or heavy chain variable region.

The invention includes modified hGP 5C4 Fab polypeptides which are functionally equivalent to hGP 5C4 Fab, or have altered but measurable hGP 5C4 Fab immunologic activity. Fragments with improved hGP 5C4 Fab immunologic activity are preferred. Examples of modified polypeptides include polypeptides with conservative substitutions of amino acid residues, and one or more deletions or additions of amino acids which do not significantly deleteriously change the functional activity.

One example is a hGP 5C4 Fab polypeptide comprising one or more amino acid substitution in comparison with the prototype hGP 5C4 Fab sequence. Substitutions can range from changing or modifying one or more amino acids to complete redesign of a region, such as the variable region. Amino acid substitutions are preferably conservative substitutions that do not deleteriously affect folding or functional properties of the peptide. Groups of functionally related amino acids within which conservative substitutions may be made are glycine/alanine; valine/isoleucine/leucine; asparagine/glutamine; aspartic acid/glutamic acid; serine/threonine/methionine; lysine/arginine; and phenylalanine/tryosine/tryptophan. Polypeptides of this invention may be in glycosylated or unglycosylated form, may be modified post-translationally (e.g., acetylation, and phosphorylation) or may be modified synthetically (e.g., the attachment of a labeling group).

The present invention also relates to humanized hGP 5C4. Since hGP 5C4 is a rodent antibody obtained from a rat, repeated *in vivo* administration in a human patient will bring about an immune response against the rodent antibody. This response may limit the effectiveness of the pharmaceutical if repeated dosing is required. The immunogenicity of the antibody may be reduced by chemical modification of the antibody with a hydrophilic polymer such as polyethylene glycol or by using the methods of genetic engineering to make the antibody binding structure more human like. For example, these procedures are described in EP 0173494, EP 194276, EP 0120694, EP 0125023, EP 0171496, EP 023940, WO 86/01533, WO 90/07861, WO91/09967 or Mol. Immunol. 28, 489 (1991).

The invention also encompasses fusion proteins comprising one or more hGP 5C4 Fab polypeptides. A hGP 5C4 Fab fusion polypeptide can be prepared, for example, by chemical synthesis, or by creating and translating a polynucleotide in which the peptide regions are encoded in the desired relationship. Alternatively, fusion proteins may be provided in expression systems constructed by co-transfection with plasmids comprising encoding regions for different functional regions of the protein.

The invention also encompasses a conjugate which comprises an effector moiety and an hGP 5C4 antibody or a function conservative fragment or variant thereof. An effector moiety may be any entity having the effect of providing an additional activity or function (e.g. a radioactivity or an enzymatic function or therapeutic activity) to the activity or function of hGP 5C4 antibody in forming the conjugate. Conjugation of the effector moiety and antibody orfunction-conservative fragment or variant thereof may be achieved by conventional methods such as chemical linkage via heterobifunctional linkers or recombinant gene fusion techniques.

The present invention also relates to a pharmaceutical composition for treatment and prevention of acute or chronic vascular diseases associated with intraarterial and/or intravenous thrombosis. The composition contains a pharmaceutically acceptable excipient and a pharmaceutically active amount of an inhibitor selected from
(a) the antibody, the function-conservative fragments or variants thereof according to the invention,
(b) the humanized antibody according to the invention,
(c) the fusion protein according to the invention, and
(d) the conjugate according to the invention.
The excipient may be a pharmaceutically-acceptable diluent or carrier. Pharmaceutical compositions of the present invention may be formulated in a variety of dosage forms. Generally, the compositions of the present invention will be administered parenterally, preferably intravenously. A particular parenteral pharmaceutical composition is formulated in a unit dosage form suitable for administration by injection. A pharmaceutically acceptable carrier or diluent may be a solid or liquid filler, diluent or substance which may be safely used in parenteral administration. Depending on the particular route of administration, a variety of pharmaceutically acceptable carriers well known in the art include solid or liquid fillers, diluents, hydrotropes, surface active agents, and encapsulating substances. The amount of carrier employed in conjunction with the inhibitor of the invention is used to provide practical quantity of material per unit dose of composition. Pharmaceutically acceptable carriers for systemic administration that may be incorporated in the composition of the invention include sugar, starches, cellulose, vegetable oils, buffers, polyols and alginic acid. Specific carriers are disclosed in US-4,401,663, EP-A 0 089 710, EP-A 0 068 592. Preferred carriers for parenteral administration include propylene glycol, pyrrolidone, ethyl oleate, aqueous ethanol, and combinations thereof. Particularly suitable compositions comprise a solution, emulsion or suspension of the inhibitor of the invention in association with a pharmaceutically acceptable parenteral carrier or diluent. Suitable carriers or diluents include aqueous vehicles, for example water or saline, and non-aqueous vehicles, for example fixed oils or liposomes. The compositions may include agents which enhance the stability of the inhibitor in the composition. For example, the composition may include a buffer such as phosphate buffered saline (PBS) containing sugars such as succrose and mannitol. The concentration of the conjugate will vary, but in general, the conjugate will be formulated at concentrations of about 1 to 10 mg/ml. The compositions may be prepared prior to use based on a freeze-dried preparation of the inhibitor.

The dose and dosage regimen will depend upon the particular inhibitor employed, the platelet population and the patient's history. The dose of the inhibitor administered will typically be in the range 0.1 to 100 mg/patient/day.

### Processes of the invention:

The present invention further relates to the use of the inhibitor of the invention for the preparation of medicament for the treatment or prevention of acute or chronic vascular diseases associated with intraarterial and/or intravenous thrombosis.

General preparation process of hGP 5C4 antibody: hGP 5C4 antibody of this invention can be prepared in several ways. It is most conveniently obtained from the hybridoma deposited as hGP 5C4 with *DSMZ-Deutsche Sammlung von Mikroorganismen und Zellkulturen* under Accession No. 2631, or the progeny thereof. For example, the cells can be cultured in a suitable medium, and spent medium can be used as an antibody source. Optionally, matrix-coated channels or beads and cell cocultures may be included to enhance growth of antibody-producing cells. For the production of large amounts of antibody, it is generally more convenient to obtain an ascites fluid. The method of raising ascites generally comprises injecting hybridoma cells into an immunologically naive histocompatible or immunotolerant mammal, especially a mouse. The mammal is optionally primed for ascites production by prior administration of a suitable composition, for example, Pristane.

hGP 5C4 may also be obtained by employing routine recombinant methods such as described in Sambrook et al. (1989). For instance, using the sequences obtainable from the hybridoma deposited with deposited with *DSMZ-Deutsche Sammlung von Mikroorganismen und Zellkulturen* under Accession No. 2631 or the progeny thereof, a polynucleotide encoding either the hGP 5C4 heavy or light chain can be cloned into a suitable expression vector (which contains control sequences for transcription, such as a promoter). The expression vector is in turn introduced into a host cell. The host cell is grown under suitable conditions such that the polynucleotide is transcribed and translated into a protein. Heavy and light chains of hGP 5C4 may be produced separately, and then combined by disulfide bond rearrangement. Alternatively, vectors with separate polynucleotides encoding each chain of hGP 5C4, or a vector with a single polynucleotide encoding both chains as separate transcripts, may be transfected into a single host cell which may then produce and assemble the entire molecule. Preferably, the host cell is a higher eukaryotic cell that can provide the normal carbohydrate complement of the molecule. The hGP 5C4 thus produced in the host cell can be purified using standard techniques in the art.

Alternatively, hGP 5C4 Fab can be chemically synthesized using information provided in this disclosure, in conjunction with standard methods of protein synthesis. A suitable method is the solid-phase Merrifield technique. Automated peptide synthesizers are commercially available, such as those manufactured by Applied Biosystems, Inc. (Foster City, Calif.).

Methods of antibody isolation are well known in the art. See, for example, Harlow and Lane (1988) Antibodies: A Laboratory Manual, Cold Spring Harbor Laboratory, New York. The hGP 5C4 antibody is a mouse immunoglobulin of the IgG class, and may be isolated by any technique suitable for immunoglobulins of this isotype. Purification methods may include salt precipitation (for example, with ammonium sulfate), ion exchange chromatography (for example, on a cationic or anionic exchange column run at neutral pH and eluted with step gradients of increasing ionic strength), gel filtration chromatography (including gel filtration HPLC), and chromatography on affinity resins such as protein A, protein G, hydroxyapatite, and anti-immunoglobulin. Most preferably, the antibody of the invention is purified by using Protein G-Sepharose columns.

The polypeptides of his invention can be made by any suitable procedure, including proteolysis of the hGP 5C4 antibody, by recombinant methods or by chemical synthesis. hGP 5C4 polypeptides, especially shorter polypeptides up to about 50 amino acids, are conveniently made by chemical synthesis, based on the information provided herein. Certain hGP 5C4 polypeptides which are fragments of the whole molecule may alternatively be prepared from enzymatic cleavage of intact hGP 5C4. Examples of proteolytic enzymes include, but are not limited to, trypsin, chymotrypsin, pepsin, papain, V8 protease, subtilisin, plasmin, and thrombin. Intact hGP 5C4 can be incubated with one or more proteinases simultaneously or sequentially. Preferably, hGP 5C4 is digested with papain for obtaining hGP 5C4 Fab. Alternatively, or in addition, intact hGP 5C4 can be treated with disulfide reducing agents. Peptides may then be separated from each other by techniques known in the art, including but not limited to gel filtration chromatography, gel electrophoresis, and reverse-phase HPLC. A hGP 5C4 polypeptide can also be made by obtaining a polynucleotide encoding it according to the information obtainable from the hGP 5C4 expressing hybridomas, and introducing it into a suitable expression system. Typically, polynucleotides encoding a hGP 5C4 Fab polypeptide are ligated into an expression vector under control of a suitable promoter and used to genetically alter the intended host cell. Both eukaryotic and prokaryotic host systems can be used. The polypeptide is then isolated from lysed cells or from the culture medium and purified to the extent needed for its intended use. Examples of prokaryotic host cells appropriate for use with this invention include E. coli. Examples of eukaryotic host cells include avian, insect, plant, and animal cells such as COS7, HeLa, and CHO cells.

For most applications, it is generally preferable that the polypeptide is at least partially purified from other cellular constituents. Preferably, the polypeptide is at least about 50% pure. as a weight percent of total protein. More preferably, the protein is at least about 50-75% pure. For clinical use, the polypeptide is preferably at least about 80% pure.

Antibody fragment hGP 5C4 Fab may be obtained by using antibody hGP 5C4 as a starting material. Specifically hGP 5C4 may be digested with a suitable proteolytic enzyme and the Fab fragments may be isolated. Preferably, hGP 5C4 is digested by using papain or a derivative thereof. Fab fragments may be purified by using affinity chromatography such as anti-immunoglobulin or ion exchange chromatography, or hydrophobic interaction chromatography.

The hGP 5C4 antibody or hGP 5C4 Fab polypeptides of this invention can be characterized in several ways. For instance, a hGP 5C4 antibody or hGP 5C4 Fab polypeptide may be tested for its ability to bind specifically to human glycoprotein VI, for its ability to specifically inhibit the binding between human glycoprotein VI and intact hGP 5C4 Fab, or for its ability to specifically inhibit the binding between human glycoprotein VI and subendothelial collagen. hGP 5C4 antibody or hGP 5C4 Fab polypeptides can also be tested for their ability to treat or prevent cardiovascular disease. hGP 5C4 antibody or hGP 5C4 Fab polypeptides can also be tested for their ability not to activate the GPVI receptor by intrinsic antibody activity or not to induce immuno-thrombocytopenia. Moreover, hGP 5C4 antibody or hGP 5C4 Fab polypeptides can also be tested for the inhibition of the release mechanism of platelets and the lack of expression of pro-inflammatory responses.

The ability of a hGP 5C4 Fab polypeptide to bind human glycoprotein VI may be tested by immunoassay. Any form of direct binding assay is suitable. In one such assay, the human glycoprotein VI or alternatively the hGP 5C4 Fab polypeptide is labeled. Suitable labels include radioisotopes such as ¹²⁵I, enzymes such as peroxidase, fluorescent labels such as fluorescein, and chemiluminescent labels. Typically, the other binding partner is insolubilized (for example, by coating onto a microtiter plate) to facilitate washing. After combining the labeled component with the insolubilized component, the solid phase is washed and the amount of bound label is determined. To conduct the inhibition assays, the putative hGP 5C4 Fab polypeptide is titered for its ability to decrease the binding of hGP 5C4 Fab to human glycoprotein VI, or human glycoprotein VI to subendothelial collagen. Either of the binding pairs in the reaction to be inhibited is labeled, while the other is typically insolubilized in order to facilitate washing. Polypeptides with the characteristics of hGP 5C4 Fab will proportionately decrease the amount of label attached to the solid phase, compared with control polypeptides. Any other characterization may be carried out as specifically described in the examples.

If hGP 5C4 Fab is to be administered to an individual, it is preferably at least 80% pure, more preferably it is at least 90% pure, even more preferably it is at least 95% pure and free of pyrogens and other contaminants. In this context, the percent purity is calculated as a weight percent of the total protein content of the preparation, and does not include constituents which are deliberately added to the composition after the hGP 5C4 Fab is purified.

The present invention provides a method of treatment of a human or animal body in need of treatment or prevention of acute or chronic vascular diseases associated with intraarterial and/or intravenous thrombosis, which comprises administration to a human or animal of a pharmaceutically effective amount of an inhibitor of the invention. If hGP 5C4 antibody or hGP 5C4 Fab are used as a medicament, the dosage will usually be in the range of from 0.1 to 100 mg/patient/ day. Preferably, hGP 5C4 antibody or hGP 5C4 Fab are used as lyophilised powders solubilised in PBS/succrose/manitol-buffer prior to parenteral administration. A human or animal body in need of treatment or prevention of acute or chronic vascular diseases associated with intraarterial and/or intravenous thrombosis is characterized by active lesions due to unmasking of subendothelial matrix collagens and platelet activation. The occurrence of such lesions can be investigated e.g. by intravascular ultrasound or thermography (e.g., Fayed and Fuster, Clinical imaging of the high-risk or vulnerable atherosclerotic plaque. Circulation 2001; 89:305-316) or nuclear resonance imaging (Helft et al., Progression and

Regression of Atherosclerotic Lesions. Circulation 2002; 105:993-998). Such lesions are highly probable in patients with acute coronary or carotid syndromes, and the risk of the reoccurrence of acute clinical complications such as myocardial infarction or stroke is very high, decreasing progressively with increasing time distance from the primary event.

### Material and Methods

*Generation of monoclonal antibodies against human GPVI.* Monoclonal antibodies were generated essentially as described (Kremmer, E., Kranz, B. R., Hille, A., Klein, K., Eulitz, M., Hoffmann-Fezer, G., Feiden, W., Herrmann, K., Delecluse, H. J., Delsol, G., Bornkamm, G. W., Mueller-Lantzsch, N., Grassert, F. A. (1995) Rat monoclonal antibodies differentiating between the Epstein-Barr virus nuclear antigens 2A (EBNA2A) and 2B (EBNA2B). *Virology* 208, 336-342). Lou/C rats were immunized with human dimeric Fc-GPVI-nt fusion protein having the amino acid sequence as shown in Figure 10. Screening of hybridoma supernatants was performed in a solid-phase immunoassay using dimeric Fc-GPVI-nt or Fc lacking the GPVI domain. Screening identified the supernatant of hybridoma different antibodies to bind specifically to dimeric Fc-GPVI-nt but not to Fc lacking the external GPVI domain. The immunoglobulin type was determined with rat Ig class (anti-IgM) and IgG subclass-specific mouse mAbs. The monoclonal antibodies were purified using Protein G-Sepharose columns. Antibody specificity of hGP 5C4 was verified by immunoblotting against dimericFc-GPVI-nt and control Fc. hGP 5C4 monoclonal antibody detected recombinant dimeric Fc-GPVI-nt but not control Fc (see **figure. 1a,** top). Furthermore, hGP 5C4 binds specifically to the surface of human platelets (see **figure 1a,** bottom).

*Generation of Fab-fragments of monoclonal IgG antibodies.* Complete IgG antibodies were digested to generate Fab-fragments of anti-GPVI antibodies with ImmunoPure Fab Kit (Pierce Biotechnology, Inc., Rockford, IL, USA) according to the manufacturer's instructions. Accordingly, IgG molecules were digested into Fab fragments and Fc fragments by using immobilized papain. After digestion, the fragments were purified on an immobilized Protein A column. Detailed instructions allow for flexibility in the protocol for hard to digest antibodies. The success of Fab-fragment generation was tested by comparing molecular size of both antibody formats in SDS gels and staining with Coomassie blue (see figure 1 b).

*Cloning of the fully human fusion protein of GPVI (Fc-GPVI-nt).* To generate a soluble form of human GPVI, the extra-cellular domain of human GPVI was cloned and fused to the human immunoglobin Fc-domain. The Fc was amplified from a human heart cDNA library (Clonetech, Palo Alto, CA) by PCR using the forward primer 5'-cgcggggcggccgcgagt-ccaaatcttgtgacaaaac-3' and the reverse primer 5'-gcgggaagctttcatttacccggagacagggag-3'. The PCR reaction was performed at 58°C annealing temperature and 20 cycles with the Expand High Fidelity PCR System (Roche Molecular Biochemicals, Mannheim, Germany). The PCR fragment was cloned in the plasmid pADTrack CMV with Notl/Hindlll and the sequence was checked by sequencing (MediGenomix, Martinsried, Germany).

For cloning of the extracellular domain of the human GPVI RNA from cultured megakaryocytes was isolated (RNeasy Mini Kit; Qiagen, Hilden, Germany) according to the manufacturer's protocol and reverse transcription was performed (Omniscript RT Kit; Qiagen) with 2µg RNA at 37°C overnight. 100 ng of the reaction was used as a template in PCR amplification of the hGPVI with the primer 5'-gcggggagatctaccaccatgtctccatccccgacc-3' and 5'-cgcggggcggccgccgttgcccttggtgtagtac-3'. The PCR reaction was performed at 54°C annealing temperature and 24 cycles with the Expand High Fidelity PCR System (Roche Molecular Biochemicals, Mannheim, Germany). The PCR fragment was cloned in the plasmid pADTrack CMV Fc with Bglll/Notl and the sequence was checked by sequencing.

*Cloning of stable Fc-GPVI-nt-CHO-Flp-in cells for expression and secretion of Fc-GPVI-nt.* The human Fc-GPVI-nt was amplified from the plasmid pADTrackCMV human Fc-GPVI-nt by PCR using the forward primer 5'- gcgggggctagcaccaccatgtctccatccccgac -3' and the reverse primer 5'- cgcgggggatcctcatttacccggagacagggag -3'. The PCR reaction was performed at 58°C annealing temperature and 24 cycles with the Expand High Fidelity PCR System (Roche Molecular Biochemicals, Mannheim, Germany). The PCR fragment was cloned in the plasmid pREP4 (Invitrogen, Carlsbad, CA) with NheI/BamHI and the sequence of the resulting plasmid pREP4 human Fc-GPVI-nt was checked by sequencing (MediGenomix, Martinsried, Germany). CHO K1 cells (DSMZ, Braunschweig, Germany) were transfected with the plasmid pREP4 human FC-GPVI-NT using effectene transfection reagent (Qiagen, Hilden, Germany). 48 hours after transfection the cells were split in medium containing 200 µg/ml hygromycin. Single colonies were picked and the expression was tested by precipitation of the recombinant protein with Protein-A-sepharose (Amersham Pharmacia Biotech AB, Uppsala, Sweden) from 1 ml culture supernatant. After SDS-PAGE the proteins were detected with peroxidase-conjugated goat anti-human IgG antibody (Fc- fragment specific; 109-035-098; Dianova, Hamburg, Germany). Fc-GPVI-nt and control Fc were expressed as secreted soluble proteins using the CHO cell line to prevent misfolding and non-glycosylation of the expressed proteins.

*Generation of stable GPVI-expressing GPVI-CHO-Flp-In cells.* The Flp-in system (Invitrogen, Karlsruhe, Germany) was used to generate a stable GPVI expressing GPVI-CHO-Flp-In cell line. In brief, full length human GPVI was cloned into the pcDNA5/FRT vector. Thereafter, 0.9 µg of GPVI-pcDNA5/FRT were co-transfected with 10 µg of pOG44, encoding the Flp recombinase, into the CHO-Flp-On cells using LipofectAMINE^{tm} together with the Plus^{tm} reagent (Invitrogen). Transfected cells were selected in medium containing 0.1 mg/ml hygromycin B.

Expression of human GPVI was confirmed by FACS analysis using anti-GPVI monoclonal antibodies. Non-transfected CHO-Flp-In cells served as controls.

*Fc-GPVI-nt protein and Fc control fully human fusion protein purification.* The culture supernatant of Fc-GPVI-nt CHO cells was collected, then applied onto two sequential tangential flow filtration devices using hollow fibre modules at a perfusion/recirculation rate of 10 I/min. A first purification was achieved via a filter from MembraPure, Bodenheim, # 5421534 (Typ Minikross, PEF, 0.5 µm pore width; 4000 ca^{m} area). Then, the filtrate was concentrated 10-fold via a second module (MembraPure # 5422532; 50 kDa pore width; 3900 cm²). The Fc-GPVI-nt fusion protein was centrifuged (3800 g, 30 min, 4°C) filtrated (0.45 µm) and precipitated by addition of 1 vol. ammonium sulphate (761 g/I) and stirred overnight at 4°C. The proteins were pelleted by centrifugation (3000 g, 30 min, 4°C), dissolved in 0.1 Vol. PBS and dialysed in PBS overnight at 4°C. Benzonase™ (Merck, cat. no 101695) is added at a concentration of 5 ng/ml to the concentrated and filtrated supernatant to brake down chromosomal DNA into oligonucleotides. The protein solution was clarified by centrifugation (3000 g, 30 min, 4°C) and loaded on a protein A column MabSelect (Amersham Pharmacia Biotech AB, Uppsala, Sweden). Some column volumes (cv) of elution buffer, 100 mM Na-citrate pH 3.2- 4.2 are run into the column mainly to be sure that there is no residual protein left from previous runs in the column. The sample is applied at a flow rate of 5 ml/min corresponding to a linear flow of 150 cm/h. The column was washed with binding buffer (20 mM sodium phosphate buffer pH 7.0, 0.02% NaN₃) until OD₂₈₀ < 0.01 and eluted with elution buffer (100 mM glycine pH 2.7). The eluted fractions were neutralized with neutralisation buffer (1 M Tris/HCl pH 9.0, 0.02 % NaN₃). Thereafter, an additional chromatographic column with a cation exchange column, Source 30 S^{TM} (Amersham BioSciences, cat. no 17-1273-01) was introduced. With an increasing salt gradient (10- 15 cv) Fc-GPVI-nt is eluted out of the column. Fractions of 1 ml are collected and appropriately pooled, dialysed in PBS overnight at 4°C, aliquoted and frozen at -20°C.

*Assessment of dimeric Fc-GPVI-nt binding to immobilized collagen.* The binding of dimeric Fc-GPVI-nt to immobilized collagen was determined. ELISA plates (Immulon2 HB, Dynx Technologies, Chantilly, VA) were coated over night at 4°C with 1 µg collagen (type I bovine; BD Bioscience, Bedford, MA) in 100 µl coating buffer (1.59 g/I Na₂CO₃, 2.93 g/l NaHCO₃, 0.2 g/l NaN₃, pH 9.6). The plates were washed with 250 µl/well PBS/0.05 % Tween 20 (PBST) twice and blocked with 250 µl/well Roti-Block (Roth, Karlsruhe, Germany) over night. The plates were washed with 250 µl/well PBST twice, then Fc-GPVI-nt in PBST was added and the plate was incubated for 1 hr at room temperature. Where indicated, Fc-GPVI-nt (20 µg/ml) was pre-incubated for 10 min with different Fab fragments of antibodies e.g. hGP 5C4 Fab (20 µg/ml) to determine inhibition of collagen - GPVI interaction. After incubation the plates were washed 5 times with 250 µl PBST and peroxidase-conjugated goat anti-human IgG antibody Fcg fragment specific (#109-035-098; Dianova, Hamburg, Germany) was added in a dilution of 1:10.000 and incubated for 1 hr at room temperature. After 5 fold washing with 250 µl PBST 100 µl detection reagent (BM Blue POD Substrate; Roche, Mannheim, Germany) was added and incubated up to 10 min. The reaction was stopped by the addition of 100 µl 1 M H₂SO₄ and the plate was measured at 450 nm against reference wavelength 690 nm.

*FACS measurement for anybody binding to GPVI-expressing CHO cells or native human platelets.* GPVI-expressing CHO cells were generated as described above. Human citrate blood was collected from volunteers Platelet rich plasma (PRP) was generated after centrifugation and washing procedures (PBS 1 x; pH 7.2) with 2000 rpm at 4°C and resuspension. GPVI-expressing CHO or control CHO cells were incubated with different antibodies where appropriate. Similarly, human platelets where incubated with different antibodies. Thereafter, secondary anti rat IgG antibodies labelled with peroxidase (Immunotech) were added. FACS measurement was performed and specific mean peroxidase fluorescence was counted with a Becton Dickenson FACScalibur device.

*FACS measurement for stimulation of different activation markers on platelets.* Human citrate blood was collected from volunteers. PRP was generated as described and diluted in staining buffer (1 x PBS (w/o Ca²⁺ and Mg⁺ ) with 0,1 % sodium azide and 2% fetal bovine serum ( FBS ), 2 mM CaCl₂) was incubated with bovine collagen type 1 (0; 2; 5 and 10 µg/ml; Nobis). For determination of antibody effects, PRP was incubated with various antibodies in staining buffer. Thereafter, stimulation with bovine collagen (10 µg/ml) or ADP (20 µmol/l) or TRAP (10 mmol/L) was followed. Anti CD 62P antibodies or anti PAC-1 antibodies labelled with the fluorophor peroxidase (Immunotech) were added. FACS measurement was performed with a Becton Dickenson FACScalibur device.

*Platelet aggregation and A TP release.* Platelet aggregation ex vivo and in vitro was evaluated by optical aggregometry in citrated blood samples at 37° C using a two-channel Chronolog aggregometer (Nobis, Germany). PRP was prepared as described and the final platelet count was adjusted to 2 x 10⁸ platelets/ml by Thyrodes-HEPES buffer (2.5 mmol/I HEPES, 150 mmol/l NaCl, 12 mmol/I NaHCO₃, 2,5 mmol/I KCI, 1 mmol/l MgCl₂, 2 mmol/I CaCl₂, 5,5 mmol D-Glucose, 1 mg/ml BSA, pH 7.4). Chrono-Lume #395 (Chrono-Log Corporation) was added for ATP measurement. For determination of antibody effects, PRP was incubated with various antibodies in different concentrations as indicated. Thereafter, agonists were added to the platelets, pipetted into the aggregometer and aggregation was started under defined stirring conditions. Aggregation was determined by change of light transmission due to coagulating platelets and normalised to an internal standard. ATP release is determined at the characteristic wavelength of Chrono-Lume for ATP and normalised to an internal standard according to the manufacturer's instructions.

*Effect of various antibodies on bleeding time of human whole blood ex vivo.* In vitro bleeding time was determined with a PFA-1 00 device (Dade-Behring). 800 µl of human whole blood was injected in the PFA-100 device. Bleeding time was measured with ADP/collagen and epinephrine/collagen coated measuring cells according to the manufacturer's instructions.

### Examples

### Specificity of antigen binding of hGP 5C4

The recognition of GPVI as an antigen for hGP 5C4 was investigated. Recombinant GPVI protein as dimeric fusion protein of the extracellular domain of the GPVI receptor and the Fc part of a human IgG1 as a linker was generated as described. Soluble GPVI protein was secreted from Fc-GPVI-nt expressing CHO cells as dimer and purified. The identity of Fc-GPVI-nt was tested in a SDS gel with Coomassie stain for proteins and with an antibody directed against the Fc part of Fc-GPVI-nt (see **figure 1a,** top). hGP 5C4 recognised the Fc-GPVI-nt specifically as purified protein in a Western blot. Under reducing conditions hGP 5C4 recognized a protein with approximately 80 kDalton (see **figure 1a**). A specific protein was also recognised from platelet lysates with approximately 65 kDalton. There was no cross reaction to the control protein Fc-control. Generation of Fab fragments of the IgG antibody hGP 5C4 was tested with SDS-PAGE and Coomassie staining (see **figure 1b**).

### Inhibition of collagen interaction with GPVI

To further address the characteristics of the anti-GPVI antibodies, we tested the ability of various antibodies to compete with immobilized collagen for the association with dimeric Fc-GPVI-nt. Only hGP 5C4 Fab inhibited Fc-GPVI-nt-dimer binding to immobilized collagen (see **figure. 2**). A concentration of 20 mg/ml antibody was required to reduce Fc-GPVI-nt-dimer binding. The antibody 4C9 Fab had no influence on collagen interactions with Fc-GPVI-nt. Together these data indicate that the novel antibody Fab fragment hGP 5C4 specifically inhibits Fc-GPVI-nt-dimer binding to collagen in vitro.

### Specific binding of anti-GPVI antibodies to GPVI expressed on CHO cells and native GPVI on human platelets

Binding of various anti-GPVI antibodies to GPVI-expressing CHO cells was tested. To further substantiate the specificity of anti-GPVI antibodies, we generated the GPVI-expressing GPVI-Flp-In^{ô}-CHO cell line. Platelets and GPVI-CHO transfectants expressed GPVI at roughly the same density as determined by flow cytometry (not shown). GPVI-transfectants but not control CHO cells avidly bound the antibodies 4C4; hGP 5C4 and 4C9 (see figure 3). The antibody clones 14E11 and CD3 did not show specific binding to GPVI-expressing CHO cells. Moreover, antibody binding to native GPVI on human platelets was tested with different anti-GPVI antibodies. In accordance, hGP 5C4 showed strong binding to native platelets with weaker binding of 4C4 and 4C9. 14E11 and CD3 did not show increased binding to platelets compared to the control antibody (see figure 4). This further supports the concept that hGP 5C4 shows specific binding to native GPVI on the surface of either GPVI-expressing CHO cells or native human platelets.

### Inhibition of platelet activation by anti-GPVI antibodies determined by FACS.

The inhibition of human platelet activation by various anti-GPVI antibodies was determined by FACS measurement of activation specific platelet markers. PAC-1 activation in human platelets was determined in presence of 4C9 Fab and hGP 5C4 Fab. hGP 5C4 Fab had no intrinsic activity for PAC-1 expression in unstimulated human platelets, whereas 4C9 Fab leads to a small activation of PAC-1 (see **figure 5a**). Stimulation with bovine collagen (10 µg/ml) activated PAC-1, which could be abolished by hGP 5C4 (20 µg/ml) but not by 4C9 Fab. Collagen (bovine type I; 10 µg/ml) typically activates human platelets and leads to increased surface expression of CD 62-P. Different anti-GPVI antibodies were tested and 4C4 Fab and hGP 5C4 Fab inhibited the collagen-mediated CD 62P activation (please see **figure 5b**). In contrast 4C9 had a potent activating effect on CD 62P in human platelets. Thus, hGP 5C4 inhibits collagen-mediated platelet activation without intrinsic activity.

Specificity of antibodies was tested for ADP-mediated and TRAP-mediated platelet activation determined by FACS. ADP (20 µmol/L) and TRAP (25µmol/l) stimulated both CD 62P (see **figure 6a**) and PAC-1 (see **figure 6b**)**.** Increasing concentrations of hGP 5C4 Fab (0.5 µg/ml to 5 µg/ml) had no effect on both ADP- and TRAP-mediated CD 62 P- and PAC-1-expression. Moreover, specificity of hGP 5C4 Fab for activation of CD63 was determined (see figure 6c). Whereas hGP 5C4 inhibited collagen-mediated CD63 activation, ADP- and TRAP-mediated CD63 was unaffected. This further supports the notion of specific inhibition of collagen-mediated platelet activation by hGP 5C4 leaving other important platelet stimulation pathways unaltered. Moreover, unchanged basal activity underlines the absence of intrinsic activity of 5C9.

### Inhibition of human platelet aggregation and A TP release byhGP 5C4 Fab.

Human platelets were pre-incubated with increasing concentrations of hGP 5C4 Fab (0.1 µg/ml to 2.0 µg/ml). Aggregation of platelets was induced by bovine collagen type I (3 µg/ml) and determined with an aggregometer under stirring conditions (for details see Material and Methods). hGP 5C4 Fab potently inhibited human platelet aggregation [in % of internal standard] ex vivo with an IC50 value of 1,2 x 10⁻⁷ g/ml (see **figure 7a**). In parallel ATP release [in % of control ATP release] was also induced by collagen and potently inhibited by hGP 5C4 Fab (0.1 µg/ml to 2.0 µg/ml) (see **figure 7b**). In contrast ADP- (5µM) or TRAP- (10 µM) mediated platelet aggregation was not affected by preincubation with significantly higher hGP 5C4 Fab concentrations (2 µg/ml and 6 µg/ml) (see **figure 8a**). hGP 5C4 inhibited collagen-mediated ATP release but had no effect on thrombin/TRAP-mediated ATP release even at higher concentrations (2 µg/ml and 6 µg/ml). ADP-mediated ATP release, however, was also inhibited by the maximal hGP 5C4 Fab concentrations (6 µg/ml) (see **figure 8b**). This also underlines the specificity of inhibition of collagen-mediated platelet aggregation of hGP 5C4 Fab. Moreover, the release of potent mediator substances from intracellular stores was also potently and selectively inhibited by hGP 5C4.

### Effect of antibodies on bleeding time of human blood ex vivo (PFA-100).

Bleeding times as the major side-effect of platelet inhibition was assessed in human whole blood ex vivo. Human platelets were stimulated with ADP/collagen or epinephrine/collagen-coated plates in a PFA-100 device. Incubation of human whole blood with hGP 5C4 Fab (5 µg/ml) had no effects on bleeding time ex vivo. In contrast, comparable and therapeutically relevant doses of ReoPro^{R} (5 µg/ml) markedly prolonged bleeding time beyond the maximum of 300 sec in the PFA-100 device (Please see **figure 9a**). Different anti-GPVI-antibodies in different antibody formats were also tested for the prolongation of bleeding time. hGP 5C4 Fab (1 µg/ml and 5 µg/ml) did not show any prolongation of bleeding time, whereas the IgG format of hGP 5C4 prolonged bleeding time in low concentrations (1 µg/ml). In contrast, 4C9 markedly prolonged bleeding time both as Fab and as IgG antibody (Please see **figure 9b**). These data further support the safety of hGP 5C4 Fab for the treatment of patients with potent antiplatelet effect without the serious side effect of bleeding complications.

### Deposit

A hybridoma cell line producing hGP 5C4 Fab antibody has been deposited under terms of the Budapest Treaty as hGP 5C4 with *DSMZ-Deutsche Sammlung von Mikroorganismen und Zellkulturen* on November 25, 2003 and has been given Accession No. 2631.

## Claims

1. Monoclonal antibody hGP 5C4 or function-conservative fragments or variants thereof, which specifically bind to glycoprotein VI.

2. The monoclonal antibody hGP 5C4 or function-conservative fragments or variants thereof according to claim 1, which specifically bind to human glycoprotein VI.

3. The monoclonal antibody according to claim 1, which is purified from an antibody producing cell deposited as hGP 5C4 with *DSMZ-Deutsche Sammlung von Mikroorganismen und Zellkulturen* under Accession No. 2631, or progeny thereof.

4. The monoclonal antibody or function-conservative fragment or variant thereof according to claim 1, 2 or 3, which inhibits collagen-binding to human glycoprotein VI.

5. Fab fragment of the monoclonal antibody according to any one of claims 1 to 4, which is hGP 5C4 Fab obtainable by papain digestion of hGP 5C4.

6. Humanized antibody specifically binding to glycoprotein VI, which comprises a function conservative fragment or variant of the monoclonal antibody hGP 5C4.

7. Fusion protein specifically binding to glycoprotein VI, which comprises an amino acid sequence of the antibody hGP 5C4 or of function-conservative fragments or variants thereof.

8. Conjugate comprising an effector moiety and the monoclonal antibody hGP 5C4 or function-conservative fragments or variants thereof, which specifically bind to glycoprotein VI as defined in any one of claims 1 to 5, or the humanized antibody of claim 6 or the fusion protein as defined in claim 7.

9. A hybridoma cell line deposited as hGP 5C4 with *DSMZ-Deutsche Sammlung von Mikroorganismen und Zellkulturen* under Accession number 2681, or a progeny thereof.

10. A nucleic acid coding for monoclonal antibody hGP 5C4 Fab or function-conservative fragments or variants thereof, wherein said nucleic acid is obtainable from the hybridoma cell line according to claim 9.

11. A nucleic acid coding for a polypeptide comprising at least 5 consecutive amino acids of the the monoclonal antibody hGP 5C4 Fab and binding specifically to human glycoprotein VI.

12. A composition comprising a pharmaceutically acceptable excipient and a pharmaceutically active amount of an inhibitor selected from
(a) the antibody, the function-conservative fragment or variant thereof according to any one of claims 1 to 5, or
(b) the humanized antibody according to claim 6,
(c) the fusion protein according to claim 7, and
(d) the conjugate according to claim 8.

13. Use of an inhibitor selected from
(a) the antibody, the function-conservative fragment or variant thereof according to any one of claims 1 to 5, or
(b) the humanized antibody according to claim 6, or
(c) the fusion protein according to claim 7, or
(d) the conjugate according to claim 8,
for the prevention or treatment of acute or chronic vascular diseases associated with intraarterial and/or intravenous thrombosis.

14. The use according to 13, wherein the medicament is for parenteral administration.

15. A process for producing monoclonal antibody hGP 5C4 or function-conservative fragments thereof, said process comprising
(i) culturing hybridoma hGP 5C4 according to claim 9, or progeny thereof in medium under conditions conducive to expression of antibody therefrom and;
(ii) obtaining antibody hGP 5C4 from the culture medium and optionally;
(iii) preparing a Fab fragment of antibody hGP 5C4 by enzymatic digestion.

16. A process for the preparation of hGP 5C4 Fab, said process comprising the step of digesting hGP 5C4 antibodies and isolating the Fab fragments.

17. The process according to claim 15, wherein hGP 5C4 is digested by using papain or a derivative thereof.
